# EUROPÄISCHE PATENTSCHRIFT

(11) **EP 3 237 067 B1**
(45) Veröffentlichungstag und Bekanntmachung des Hinweises auf die Patenterteilung: **23.10.2019**
(21) Anmeldenummer: 15794495.0
(22) Anmeldetag: 02.11.2015
(51) Int. Cl.: A61Q 5/08, A61Q 5/10, A61K 8/34, A61K 8/37, A61K 8/39, A61K 8/86

(54) **GERUCHSREDUZIERENDER ENTWICKLER FÜR OXIDATIONSFÄRBEMITTEL**
ODOR-REDUCING DEVELOPER FOR OXIDATION DYES
DÉVELOPPEUR RÉDUCTEUR D'ODEURS POUR COLORANTS D'OXYDATION

(30) Priorität: 22.12.2014 DE 102014226747
(43) Veröffentlichungstag der Anmeldung: 01.11.2017
(73) Patentinhaber: Henkel AG & Co. KGaA, 40589 Düsseldorf (DE)
(72) Erfinder: MANNECK, Hartmut, 23858 Barnitz (DE); HOEPFNER, Stefan, 22523 Hamburg (DE); SCHWEINSBERG, Matthias, 40764 Langenfeld (DE); KLEEN-FEHRES, Astrid, 20457 Hamburg (DE)
(86) Internationale Anmeldenummer: PCT/EP2015/075430
(87) Internationale Veröffentlichungsnummer: WO 2016/102107

(56) Entgegenhaltungen:
- WO-A2-2014/090645
- WO-A2-2015/018412

## Beschreibung

Die vorliegende Anmeldung betrifft Zusammensetzungen, die sich als Oxidationsmittel für Seifengelbasierte Haaraufhell- und/oder Haarfärbezubereitungen eignen, wobei die Seifengelbasierten Haaraufhell- und/oder Haarfärbezubereitungen Ammoniak bzw. Ammoniumhydroxid als Alkalisierungsmittel und gegebenenfalls mindestens ein Oxidationsfarbstoffvorprodukt enthalten und einen pH-Wert im Bereich von 8 bis 11,5, gemessen bei 20°C, aufweisen.

Mit den erfindungsgemäßen Oxidationsmitteln lassen sich die die Anwendungseigenschaften der Anwendungsmischung, insbesondere die Freisetzung von und der Geruch nach Ammoniak deutlich reduzieren.

Ein weiterer Gegenstand der vorliegenden Anmeldung betrifft Mittel zur Farbveränderung keratinischer Fasern, die aus zwei voneinander getrennten Zusammensetzungen durch Vermischen der beiden Zusammensetzungen herstellbar sind, wobei eine der beiden Zusammensetzungen eine oxidative Zusammensetzung gemäß dem ersten Gegenstand der Anmeldung und die zweite Zusammensetzung eine Seifengelbasierte Haaraufhell- und/oder Haarfärbezubereitung ist, die Ammoniak als Alkalisierungsmittel und gegebenenfalls mindestens ein Oxidationsfarbstoffvorprodukt enthält und einen pH-Wert im Bereich von 8 bis 11,5, gemessen bei 20°C, aufweist und weiterhin bevorzugt, jeweils bezogen auf ihr Gewicht, 25 - 85 Gew.-% Wasser, weiterhin 5-20 Gew.-%, bevorzugt 8 - 16 Gew.-% mindestens eines Salzes einer C12-C22-Fettsäure, bevorzugt eines Salzes der Ölsäure, optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül, 2-20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül, insgesamt 0 - 1 Gew.-% mindestens eines Öls, bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-% und gegebenenfalls mindestens ein Oxidationsfarbstoffvorprodukt, wobei alle Mengenangaben auf das Gewicht der Zusammensetzung (A) bezogen sind, enthält.

Ein weiterer Gegenstand der vorliegenden Anmeldung betrifft ein Kit zur oxidativen Farbveränderung keratinischer Fasern, umfassend eine Seifengelbasierte, ggf. Farbstoff-haltige, Ammoniak-haltige Alkalisierungszubereitung und eine wässrige Wasserstoffperoxidzubereitung, wobei die Wasserstoffperoxidzubereitung dahingehend optimiert ist, dass die gebrauchsfertige Mischung aus Alkalisierungsgel und Wasserstoffperoxidzubereitung eine viskose Creme oder Paste mit einer Viskosität im Bereich von 2000 bis 5000 mPas (beispielsweise gemessen bei 20°C mit einem Haake-Viskosimeter Typ MV2 mit einer Geschwindigkeit von 8 Umdrehungen/ Minute) darstellt, die sich gut auf die aufzuhellenden und/oder zu färbenden Fasern auftragen lässt und während der Anwendungsdauer von 5 bis 60 Minuten eine verringerte Ammoniak-Freisetzung aufweist.

Ein weiterer Gegenstand der vorliegenden Anmeldung betrifft ein Verfahren zur oxidativen Farbveränderung keratinischer Fasern, wobei das anwendungsbereite Aufhell- und/oder Färbemittel durch Vermischen der Komponenten des vorgenannten Kits unmittelbar vor der Anwendung hergestellt wird, anschließend auf die Fasern, insbesondere Haare, aufgetragen und nach einer Einwirkzeit von 5 bis 60 Minuten wieder abgespült wird.

Die vorliegende Erfindung bezieht sich auf die oxidative Farbveränderung keratinischer Fasern, insbesondere Haaren. Da bei der Behandlung von keratinischer Fasern, insbesondere Haaren, mit Oxidationsmitteln, insbesondere mit Wasserstoffperoxid, der fasereigene Farbstoff Melanin zu einem gewissen Grad zerstört wird, werden die Fasern/Haare zwangsläufig aufgehellt, ändern also ihre Farbe auch ohne die Gegenwart eines Farbstoffs. Daher umfasst der Begriff "Farbveränderung" im Sinne der vorliegenden Anmeldung sowohl die Aufhellung als auch die Färbung mit einem oder mehreren Farbstoffen.

Für die Farbveränderung von menschlichen Haaren kennt der Fachmann verschiedene Verfahren. Im allgemeinen werden für die Färbung menschlicher Haare entweder direktziehende Farbstoffe oder Oxidationsfarbstoffe verwendet, welche durch oxidative Kupplung einer oder mehrerer Entwicklerkomponenten untereinander oder mit einer oder mehreren Kupplerkomponenten entstehen. Kuppler- und Entwicklerkomponenten werden auch als Oxidationsfarbstoffvorprodukte bezeichnet. Die mit Oxidationsfarben erzielten Färbungen werden zumeist als permanente oder semipermanente Färbungen bezeichnet.

Als Oxidationsmittel enthalten diese Mittel zumeist Wasserstoffperoxid. Da Wasserstoffperoxid im alkalischen pH-Bereich nur unzureichend lagerstabil ist, bestehen oxidative Färbemittel üblicherweise aus zwei Komponenten, die unmittelbar vor der Anwendung miteinander vermischt werden. Die eine Komponente enthält Wasserstoffperoxid in wässriger Lösung oder Emulsion, wobei diese Zusammensetzung zur Stabilisierung des Wasserstoffperoxids einen sauren pH-Wert im Bereich von 2,5 bis 5,5 aufweist. Die zweite Komponente enthält ein oder mehrere Alkalisierungsmittel in einer solchen Menge, dass die Anwendungsmischung aus beiden Komponenten einen pH-Wert im Bereich von 8 bis 11 aufweist. Sofern die Alkalisierungszubereitung keinen Farbstoff oder nur geringe Mengen an Farbstoff enthält - letzteres dient zur Kaschierung unerwünschter Farbtöne, die bei der Melaninoxidation entstehen können -, handelt es sich um ein Aufhell- oder Bleichmittel. Die Alkalisierungszubereitung kann aber auch Oxidationsfarbstoffvorprodukte und/oder direktziehende Farbstoffe enthalten; die resultierende Anwendungsmischung dient dann als Färbemittel. Daneben gibt es auch Färbekits und Färbeverfahren, bei denen die Anwendungsmischung aus beiden Komponenten einen pH-Wert im Bereich von etwa 6 bis 7,9 aufweist; die Färbeergebnisse dieser so genannten "sauren" Färbungen erreichen allerdings häufig nicht die Güte, die mit alkalischen Anwendungsmischungen erzielt werden.

Zur oxidativen Haarfarbveränderung kommen, insbesondere auf dem nordamerikanischen Markt, bevorzugt Ammoniak-haltige Alkalisierungszubereitungen zum Einsatz, die als Seifenbasiertes Gel vorliegen und insbesondere größere Mengen an Seifen, das heißt, Salzen von Fettsäuren, und ggf. anderen Tensiden, aber beispielsweise kaum oder nur wenig Fettalkohole enthalten. Derartige Rezepturen weisen im Vergleich zu Fettalkohol-haltigen alkalischen Blondier- oder Färbecremes bei der Anwendung auf dem Haar einen sehr starken Geruch nach Ammoniak auf.

Weiterhin enthalten die Alkalisierungszubereitungen Ammoniak als Alkalisierungsmittel und gegebenenfalls mindestens ein Oxidationsfarbstoffvorprodukt und weisen einen pH-Wert im Bereich von 8 bis 11,5, gemessen bei 20°C, auf.

Weiterhin werden bevorzugt Alkalisierungszubereitungen eingesetzt, die, jeweils bezogen auf ihr Gewicht, 25 - 85 Gew.-% Wasser, weiterhin 5-20 Gew.-%, bevorzugt 8 - 16 Gew.-% mindestens eines Salzes einer C12-C22-Fettsäure, bevorzugt eines Salzes der Ölsäure, optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül, 2-20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül, insgesamt 0 -1 Gew.-% mindestens eines Öls, bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-% und gegebenenfalls mindestens ein Oxidationsfarbstoffvorprodukt, wobei alle Mengenangaben auf das Gewicht der Zusammensetzung (A) bezogen sind,enthalten.

Zur oxidativen Haarfarbveränderung wird üblicherweise die Alkalisierungszubereitung mit einer wässrigen Oxidationsmittelzubereitung vermischt, beispielsweise in einer wiederverschließbaren Flasche oder einem Schüttelbecher, und die daraus resultierende Anwendungsmischung wird auf das zu behandelnde Haar aufgetragen, wo sie für eine Einwirkzeit von 5 bis 60 Minuten verbleibt, bevor sie wieder abgespült wird.

Im Stand der Technik sind bereits diverse Lösungsansätze bekannt, um den Ammoniakgeruch eines Haarbehandlungsmittels zu reduzieren. Zum Beispiel kann an Stelle des Ammoniaks ein Alkanolamin, insbesondere Monoethanolamin, eingesetzt werden, das kaum flüchtig ist und weniger stark riecht als Ammoniak. Allerdings hat sich gezeigt, dass das Farbaufnahmevermögen der Keratinfasern am besten beim Einsatz von Ammoniak ist. Will man das geliche Farbergebnis mit Monoethanolamin erzielen, muss man mehr Wasserstoffperoxid einsetzen oder aber die Anwendungsmischung länger auf den Keratinfasern einwirken lassen. Letztendlich führt die Verwendung von Monoethanolamin also zu einer höheren Schädigung der Keratinfasern als Ammoniak.

Eine andere Möglichkeit zur Geruchsreduzierung der Anwendungsmischung ist es, mit größeren Mengen an Parfüm den Ammoniakgeruch zu kaschieren. Dies ist aufgrund des sehr stechenden Geruchs von Ammoniak allerdings nur schwierig zu erzielen.

Aufgabe der vorliegenden Erfindung war es, eine verbesserte Oxidationsmittelzubereitung für oxidative Farbveränderungsmittel, die als Seifenbasiertes Gel vorliegen und Ammoniak als Alkalisierungsmittel und gegebenenfalls mindestens ein Oxidationsfarbstoffvorprodukt enthalten und einen pH-Wert im Bereich von 8 bis 11,5, gemessen bei 20°C, aufweisen, bereitzustellen, mit denen sich homogene und viskositätsstabile Anwendungsmischungen herstellen lassen, die während der gesamten Einwirkzeit eine verringerte Ammoniakfreisetzung aufweisen.

Es wurde überraschend gefunden, dass wässrige Wasserstoffperoxid-Zubereitungen, enthaltend ausgewählte 1-Alkanole, Glycerylfettsäureester, ethoxylierte Fettalkohole, Polyole und Öle, die gestellte Aufgabe in sehr guter Weise lösen.

Ein erster Gegenstand der vorliegenden Erfindung ist daher eine Oxidationszusammensetzung zur oxidativen Haarbehandlung, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens ein lineares gesättigtes 1-Alkanol mit 12 - 30 Kohlenstoffatomen enthalten ist, bevorzugt in einer Gesamtmenge von 2 - 8 Gew.-%, besonders bevorzugt 3 bis 6,5 Gew.-%,
- mindestens einen Glycerylfettsäureester der allgemeinen Formel (I), 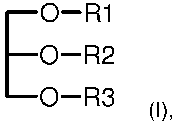 worin
- R1, R2 und R3 unabhängig voneinander für ein Wasserstoffatom oder eine Gruppierung der Formel (II) stehen, 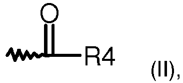 worin
- R4 für eine unverzweigte oder verzweigte, gesättige oder ungesättigte C₁₁-C₂₇-Alkylgruppe steht,
   - mit der Maßgabe, dass mindestens einer und maximal zwei der Reste ausgewählt aus R1, R2 und R3 für eine Gruppierung der Formel (II) stehen,
      wobei der mindestens eine Glycerylfettsäureester der allgemeinen Formel (I) bevorzugt in einer Gesamtmenge von 0,01 - 1 Gew.-%, besonders bevorzugt 0,1 bis 0,8 Gew.-%, enthalten ist,
- mindestens einen ethoxylierten Fettalkohol der Formel (III) 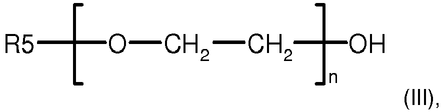 worin
   R5 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
   n für eine ganze Zahl von 10 bis 30, bevorzugt für eine ganze Zahl von 12 bis 25, weiter bevorzugt für eine ganze Zahl von 15 bis 20 steht,
   wobei der mindestens eine ethoxylierte Fettalkohol der Formel (III) bevorzugt in einer Gesamtmenge von 0,25 - 2 Gew.-%, besonders bevorzugt 0,5 bis 1,5 Gew.-%, enthalten ist,
- mindestens einen ethoxylierten Fettalkohol der Formel (IV) 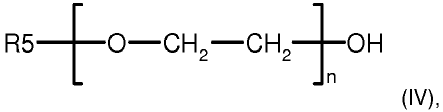 worin
   R5 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und n für eine ganze Zahl von 50 bis 150, bevorzugt für eine ganze Zahl von 80 bis 120, weiter bevorzugt für die Zahl 100 steht,
   wobei der mindestens eine ethoxylierte Fettalkohol der Formel (IV) bevorzugt in einer Gesamtmenge von 0,03 - 0,3 Gew.-%, besonders bevorzugt 0,08 bis 0,25 Gew.-%, außerordentlich bevorzugt 0,1 - 0,2 Gew.-%, enthalten ist,
- mindestens ein Polyol, ausgewählt aus C₂ - C₉-Alkanolen mit 2 - 6 Hydroxylgruppen und Polyethylenglycolen mit 3 - 20 Ethylenoxid-Einheiten sowie Mischungen hiervon, bevorzugt in einer Gesamtmenge von 1-10 Gew.-%, besonders bevorzugt 2 - 8 Gew.-%, außerordentlich bevorzugt 3 - 6 Gew.-%,
- mindestens ein Öl, bevorzugt in einer Gesamtmenge von 0,1 - 5 Gew.-%, besonders bevorzugt 0,2 - 3 Gew.-%, außerordentlich bevorzugt 0,5 - 2,5 Gew.-%,
wobei alle Mengenangaben auf das Gewicht der Oxidationszusammensetzung bezogen sind, dadurch gekennzeichnet, dass mindestens eine lamellare Phase enthalten ist.

Die erfindungsgemäße Oxidationszusammensetzung enthält, jeweils bezogen auf ihr Gewicht, 50 - 96 Gew.-%, bevorzugt 70 - 93 Gew.-%, besonders bevorzugt 80 - 90 Gew.-% Wasser.

Die erfindungsgemäße Oxidationszusammensetzung enthält, jeweils bezogen auf ihr Gewicht, 0,5 - 20 Gew.-%, bevorzugt 3 - 12 Gew.-%, besonders bevorzugt 6 - 9 Gew.-% Wasserstoffperoxid.

Die erfindungsgemäße Oxidationszusammensetzung enthält mindestens ein

Weitere erfindungsgemäß bevorzugte Oxidationszusammensetzungen sind dadurch gekennzeichnet, dass mindestens ein lineares gesättigtes 1-Alkanol mit 12 - 30 Kohlenstoffatomen enthalten ist, bevorzugt in einer Gesamtmenge von 2 - 8 Gew.-%, besonders bevorzugt 3 bis 6,5 Gew.-%, jeweils bezogen auf das Gewicht der Oxidationszusammensetzung.

Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol und Behenylalkohol sowie aus Mischungen dieser Alkanole, besonders bevorzugt aus Cetylalkohol, Stearylalkohol, Arachidylalkohol, Behenylalkohol und Cetylalkohol/ Stearylalkohol-Mischungen. Bevorzugte erfindungsgemäße Oxidationszusammensetzungen enthalten, jeweils bezogen auf ihr Gewicht, mindestens ein lineares gesättigtes 1-Alkanol mit 12 - 30 Kohlenstoffatomen in einer Gesamtmenge von 2,7 - 6 Gew.-%, bevorzugt in einer Gesamtmenge von 3,0 - 5,0 Gew.-%, wobei mindestens ein 1-Alkanol, ausgewählt aus Cetylalkohol, Stearylalkohol und Cetylalkohol/ Stearylalkohol-Mischungen, enthalten ist.

Als vierten wesentlichen Formulierungsbestandteil enthalten die erfindungsgemäßen Oxidationszusammensetzungen mindestens einen Glycerylfettsäureester der allgemeinen Formel (I), 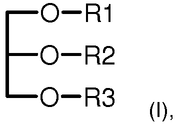 worin
R1, R2 und R3 unabhängig voneinander für ein Wasserstoffatom oder eine Gruppierung der Formel (II) stehen, worin 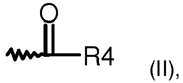 R4 für eine unverzweigte oder verzweigte, gesättige oder ungesättigte C₁₁-C₂₇-Alkylgruppe steht, mit der Maßgabe, dass mindestens einer und maximal zwei der Reste ausgewählt aus R1, R2 und R3 für eine Gruppierung der Formel (II) stehen.

Der Rest R4 in der Formel (II) steht für eine unverzweigte oder verzweigte, gesättigte oder ungesättigte C₁₁-C₂₇-Alkylgruppe.

Bevorzugt steht R4 für eine unverzweigte, gesättigte C₁₁-C₂₇-Alkylgruppe. Weiter bevorzugt steht R4 für eine unverzweigte, gesättigte C₁₃-C₂₃-Alkylgruppe. Besonders bevorzugt steht R4 für eine unverzweigte, gesättigte C₁₅-C₁₇-Alkylgruppe

Erfindungsgemäß bevorzugte Oxidationszusammensetzungen sind dadurch gekennzeichnet, dass als Glycerylfettsäureester der allgemeinen Formel (I) mindestens eine Verbindung aus der Gruppe der Formeln (Ia) bis (Id) enthalten ist:

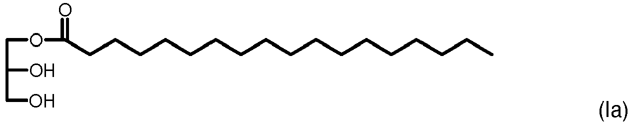

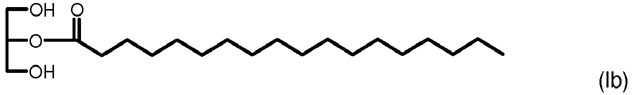

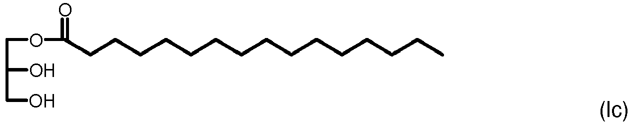

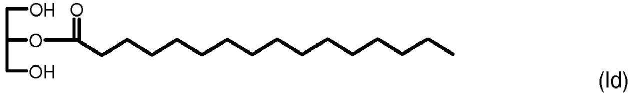

Die Verbindungen der Formeln (Ia) bis (Id) sind auch unter den Namen Glycerylmonostearat und Glycerylmonopalmitat bekannt.

Weitere erfindungsgemäß bevorzugte Oxidationszusammensetzungen sind dadurch gekennzeichnet, dass als Glycerylfettsäureester der allgemeinen Formel (I) mindestens eine Verbindung aus der Gruppe der Formeln (Ie) bis (Ih) enthalten ist:

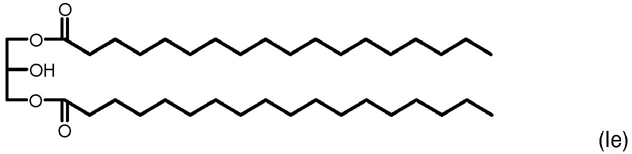

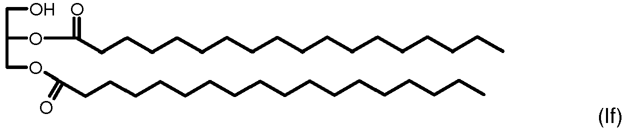

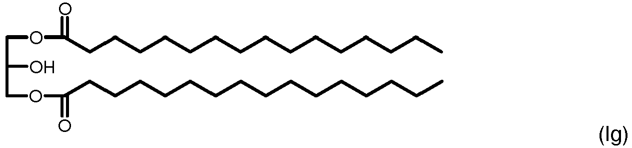

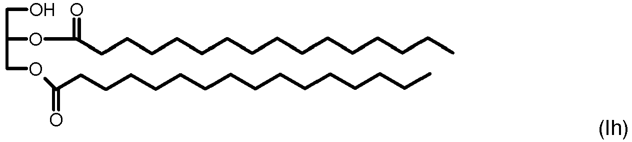

Die Verbindungen der Formel (Ie) bis (Ih) sind auch unter den Namen Glyceryldistearat und Glyceryldipalmitat bekannt.

Weitere erfindungsgemäß bevorzugte Oxidationszusammensetzungen sind dadurch gekennzeichnet, dass als Glycerylfettsäureester der Formel (I) mindestens eine Verbindung aus der Gruppe der Formeln (Ia) bis (Ih) enthalten ist.

Weitere erfindungsgemäß besonders bevorzugte Oxidationszusammensetzungen sind dadurch gekennzeichnet, dass als Glycerylfettsäureester der Formel (I) mindestens eine Verbindung, ausgewählt aus Glycerylmonostearat, Glyceryldistearat, Glycerylmonopalmitat und Glyceryldipalmitat, enthalten ist.

Die Glycerylfettsäureester der Formel (I) bewirken in Kombination mit dem mindestens einen langkettigen 1-Alkanol eine Reduktion des Ammoniakgeruchs. Die Geruchsreduktion ist besonders ausgeprägt, wenn Glycerylfettsäureester der Formel (I) und das mindestens eine langkettige 1-Alkanol in bestimmten Verhältnissen zueinander enthalten sind. Daher sind auch die Glycerylfettsäureester der Formel (I) bevorzugt in bestimmten Mengen in der erfindungsgemäßen Oxidationszusammensetzung enthalten.

Besonders bevorzugt ist es, wenn die erfindungsgemäße Oxidationszusammensetzung, bezogen auf ihr Gewicht, ein oder mehrere Glycerylfettsäureester der Formel (I) in einer Gesamtmenge von 0,01 - 1 Gew.-%, besonders bevorzugt 0,1 bis 0,8 Gew.-%, enthält.

In einer weiteren besonders bevorzugten Ausführungsform ist eine erfindungsgemäße Oxidationszusammensetzung dadurch gekennzeichnet, dass sie als Glycerylfettsäureester der Formel (I) mindestens eine Verbindung, ausgewählt aus Glycerylmonostearat, Glyceryldistearat, Glycerylmonopalmitat und Glyceryldipalmitat, in einer Gesamtmenge von 0,01 bis 1 Gew.-%, bevorzugt von 0,1 bis 0,8 Gew.-%, bezogen auf das Gewicht der erfindungsgemäßen Oxidationszusammensetzung, enthält.

Als fünften wesentlichen Formulierungsbestandteil enthalten die erfindungsgemäßen Oxidationszusammensetzungen mindestens einen ethoxylierten Fettalkohol der Formel (III) 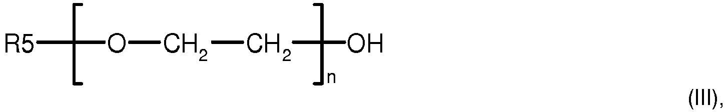 worin
R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und n für eine ganze Zahl von 10 bis 30, bevorzugt für eine ganze Zahl von 12 bis 25, weiter bevorzugt für eine ganze Zahl von 15 bis 20 steht, wobei der mindestens eine ethoxylierte Fettalkohol der Formel (III) bevorzugt in einer Gesamtmenge von 0,25 - 2 Gew.-%, besonders bevorzugt 0,5 bis 1,5 Gew.-%, enthalten ist.

Ganz besonders bevorzugt ist es, wenn als ethoxylierter Fettalkohol der Formel (III) ein oder mehrere ethoxylierte Fettalkohole aus der Gruppe
- Decan-1-ol (Decylalkohol), ethoxyliert mit 10 EO,
- Decan-1-ol (Decylalkohol), ethoxyliert mit 11 EO,
- Decan-1-ol (Decylalkohol), ethoxyliert mit 12 EO,
- Decan-1-ol (Decylalkohol), ethoxyliert mit 13 EO,
- Decan-1-ol (Decylalkohol), ethoxyliert mit 14 EO,
- Decan-1-ol (Decylalkohol), ethoxyliert mit 15 EO,
- Decan-1-ol (Decylalkohol), ethoxyliert mit 16 EO,
- Decan-1-ol (Decylalkohol), ethoxyliert mit 17 EO,
- Decan-1-ol (Decylalkohol), ethoxyliert mit 18 EO,
- Decan-1-ol (Decylalkohol), ethoxyliert mit 19 EO,
- Decan-1-ol (Decylalkohol), ethoxyliert mit 20 EO,
- Decan-1-ol (Decylalkohol), ethoxyliert mit 21 EO,
- Decan-1-ol (Decylalkohol), ethoxyliert mit 22 EO,
- Decan-1-ol (Decylalkohol), ethoxyliert mit 23 EO,
- Decan-1-ol (Decylalkohol), ethoxyliert mit 24 EO,
- Decan-1-ol (Decylalkohol), ethoxyliert mit 25 EO,
- Decan-1-ol (Decylalkohol), ethoxyliert mit 26 EO,
- Decan-1-ol (Decylalkohol), ethoxyliert mit 27 EO,
- Decan-1-ol (Decylalkohol), ethoxyliert mit 28 EO,
- Decan-1-ol (Decylalkohol), ethoxyliert mit 29 EO,
- Decan-1-ol (Decylalkohol), ethoxyliert mit 30 EO,
- Dodecan-1-ol (Dodecylalkohol, Laurylalkohol), ethoxyliert mit 10 EO,
- Dodecan-1-ol (Dodecylalkohol, Laurylalkohol), ethoxyliert mit 11 EO,
- Dodecan-1-ol (Dodecylalkohol, Laurylalkohol), ethoxyliert mit 12 EO,
- Dodecan-1-ol (Dodecylalkohol, Laurylalkohol), ethoxyliert mit 13 EO,
- Dodecan-1-ol (Dodecylalkohol, Laurylalkohol), ethoxyliert mit 14 EO,
- Dodecan-1-ol (Dodecylalkohol, Laurylalkohol), ethoxyliert mit 15 EO,
- Dodecan-1-ol (Dodecylalkohol, Laurylalkohol), ethoxyliert mit 16 EO,
- Dodecan-1-ol (Dodecylalkohol, Laurylalkohol), ethoxyliert mit 17 EO,
- Dodecan-1-ol (Dodecylalkohol, Laurylalkohol), ethoxyliert mit 18 EO,
- Dodecan-1-ol (Dodecylalkohol, Laurylalkohol), ethoxyliert mit 19 EO,
- Dodecan-1-ol (Dodecylalkohol, Laurylalkohol), ethoxyliert mit 20 EO,
- Dodecan-1-ol (Dodecylalkohol, Laurylalkohol), ethoxyliert mit 21 EO,
- Dodecan-1-ol (Dodecylalkohol, Laurylalkohol), ethoxyliert mit 22 EO,
- Dodecan-1-ol (Dodecylalkohol, Laurylalkohol), ethoxyliert mit 23 EO,
- Dodecan-1-ol (Dodecylalkohol, Laurylalkohol), ethoxyliert mit 24 EO,
- Dodecan-1-ol (Dodecylalkohol, Laurylalkohol), ethoxyliert mit 25 EO,
- Dodecan-1-ol (Dodecylalkohol, Laurylalkohol), ethoxyliert mit 26 EO,
- Dodecan-1-ol (Dodecylalkohol, Laurylalkohol), ethoxyliert mit 27 EO,
- Dodecan-1-ol (Dodecylalkohol, Laurylalkohol), ethoxyliert mit 28 EO,
- Dodecan-1-ol (Dodecylalkohol, Laurylalkohol), ethoxyliert mit 29 EO,
- Dodecan-1-ol (Dodecylalkohol, Laurylalkohol), ethoxyliert mit 30 EO,
- Tetradecan-1-ol, (Tetradecylalkohol, Myristylalkohol), ethoxyliert mit 10 EO,
- Tetradecan-1-ol, (Tetradecylalkohol, Myristylalkohol), ethoxyliert mit 11 EO,
- Tetradecan-1-ol, (Tetradecylalkohol, Myristylalkohol), ethoxyliert mit 12 EO,
- Tetradecan-1-ol, (Tetradecylalkohol, Myristylalkohol), ethoxyliert mit 13 EO,
- Tetradecan-1-ol, (Tetradecylalkohol, Myristylalkohol), ethoxyliert mit 14 EO,
- Tetradecan-1-ol, (Tetradecylalkohol, Myristylalkohol), ethoxyliert mit 15 EO,
- Tetradecan-1-ol, (Tetradecylalkohol, Myristylalkohol), ethoxyliert mit 16 EO,
- Tetradecan-1-ol, (Tetradecylalkohol, Myristylalkohol), ethoxyliert mit 17 EO,
- Tetradecan-1-ol, (Tetradecylalkohol, Myristylalkohol), ethoxyliert mit 18 EO,
- Tetradecan-1-ol, (Tetradecylalkohol, Myristylalkohol), ethoxyliert mit 19 EO,
- Tetradecan-1-ol, (Tetradecylalkohol, Myristylalkohol), ethoxyliert mit 20 EO,
- Tetradecan-1-ol, (Tetradecylalkohol, Myristylalkohol), ethoxyliert mit 21 EO,
- Tetradecan-1-ol, (Tetradecylalkohol, Myristylalkohol), ethoxyliert mit 22 EO,
- Tetradecan-1-ol, (Tetradecylalkohol, Myristylalkohol), ethoxyliert mit 23 EO,
- Tetradecan-1-ol, (Tetradecylalkohol, Myristylalkohol), ethoxyliert mit 24 EO,
- Tetradecan-1-ol, (Tetradecylalkohol, Myristylalkohol), ethoxyliert mit 25 EO,
- Tetradecan-1-ol, (Tetradecylalkohol, Myristylalkohol), ethoxyliert mit 26 EO,
- Tetradecan-1-ol, (Tetradecylalkohol, Myristylalkohol), ethoxyliert mit 27 EO,
- Tetradecan-1-ol, (Tetradecylalkohol, Myristylalkohol), ethoxyliert mit 28 EO,
- Tetradecan-1-ol, (Tetradecylalkohol, Myristylalkohol), ethoxyliert mit 29 EO,
- Tetradecan-1-ol, (Tetradecylalkohol, Myristylalkohol), ethoxyliert mit 30 EO,
- Hexadecan-1-ol (Hexadecylalkohol, Cetylalkohol, Palmitylalkohol), ethoxyliert mit 10 EO,
- Hexadecan-1-ol (Hexadecylalkohol, Cetylalkohol, Palmitylalkohol), ethoxyliert mit 11 EO,
- Hexadecan-1-ol (Hexadecylalkohol, Cetylalkohol, Palmitylalkohol), ethoxyliert mit 12 EO,
- Hexadecan-1-ol (Hexadecylalkohol, Cetylalkohol, Palmitylalkohol), ethoxyliert mit 13 EO,
- Hexadecan-1-ol (Hexadecylalkohol, Cetylalkohol, Palmitylalkohol), ethoxyliert mit 14 EO,
- Hexadecan-1-ol (Hexadecylalkohol, Cetylalkohol, Palmitylalkohol), ethoxyliert mit 15 EO,
- Hexadecan-1-ol (Hexadecylalkohol, Cetylalkohol, Palmitylalkohol), ethoxyliert mit 16 EO,
- Hexadecan-1-ol (Hexadecylalkohol, Cetylalkohol, Palmitylalkohol), ethoxyliert mit 17 EO,
- Hexadecan-1-ol (Hexadecylalkohol, Cetylalkohol, Palmitylalkohol), ethoxyliert mit 18 EO,
- Hexadecan-1-ol (Hexadecylalkohol, Cetylalkohol, Palmitylalkohol), ethoxyliert mit 19 EO,
- Hexadecan-1-ol (Hexadecylalkohol, Cetylalkohol, Palmitylalkohol), ethoxyliert mit 20 EO,
- Hexadecan-1-ol (Hexadecylalkohol, Cetylalkohol, Palmitylalkohol), ethoxyliert mit 21 EO,
- Hexadecan-1-ol (Hexadecylalkohol, Cetylalkohol, Palmitylalkohol), ethoxyliert mit 22 EO,
- Hexadecan-1-ol (Hexadecylalkohol, Cetylalkohol, Palmitylalkohol), ethoxyliert mit 23 EO,
- Hexadecan-1-ol (Hexadecylalkohol, Cetylalkohol, Palmitylalkohol), ethoxyliert mit 24 EO,
- Hexadecan-1-ol (Hexadecylalkohol, Cetylalkohol, Palmitylalkohol), ethoxyliert mit 25 EO,
- Hexadecan-1-ol (Hexadecylalkohol, Cetylalkohol, Palmitylalkohol), ethoxyliert mit 26 EO,
- Hexadecan-1-ol (Hexadecylalkohol, Cetylalkohol, Palmitylalkohol), ethoxyliert mit 27 EO,
- Hexadecan-1-ol (Hexadecylalkohol, Cetylalkohol, Palmitylalkohol), ethoxyliert mit 28 EO,
- Hexadecan-1-ol (Hexadecylalkohol, Cetylalkohol, Palmitylalkohol), ethoxyliert mit 29 EO,
- Hexadecan-1-ol (Hexadecylalkohol, Cetylalkohol, Palmitylalkohol), ethoxyliert mit 30 EO,
- Octadecan-1-ol (Octadecylalkohol, Stearylalkohol), ethoxyliert mit 10 EO,
- Octadecan-1-ol (Octadecylalkohol, Stearylalkohol), ethoxyliert mit 11 EO,
- Octadecan-1-ol (Octadecylalkohol, Stearylalkohol), ethoxyliert mit 12 EO,
- Octadecan-1-ol (Octadecylalkohol, Stearylalkohol), ethoxyliert mit 13 EO,
- Octadecan-1-ol (Octadecylalkohol, Stearylalkohol), ethoxyliert mit 14 EO,
- Octadecan-1-ol (Octadecylalkohol, Stearylalkohol), ethoxyliert mit 15 EO,
- Octadecan-1-ol (Octadecylalkohol, Stearylalkohol), ethoxyliert mit 16 EO,
- Octadecan-1-ol (Octadecylalkohol, Stearylalkohol), ethoxyliert mit 17 EO,
- Octadecan-1-ol (Octadecylalkohol, Stearylalkohol), ethoxyliert mit 18 EO,
- Octadecan-1-ol (Octadecylalkohol, Stearylalkohol), ethoxyliert mit 19 EO,
- Octadecan-1-ol (Octadecylalkohol, Stearylalkohol), ethoxyliert mit 20 EO,
- Octadecan-1-ol (Octadecylalkohol, Stearylalkohol), ethoxyliert mit 21 EO,
- Octadecan-1-ol (Octadecylalkohol, Stearylalkohol), ethoxyliert mit 22 EO,
- Octadecan-1-ol (Octadecylalkohol, Stearylalkohol), ethoxyliert mit 23 EO,
- Octadecan-1-ol (Octadecylalkohol, Stearylalkohol), ethoxyliert mit 24 EO,
- Octadecan-1-ol (Octadecylalkohol, Stearylalkohol), ethoxyliert mit 25 EO,
- Octadecan-1-ol (Octadecylalkohol, Stearylalkohol), ethoxyliert mit 26 EO,
- Octadecan-1-ol (Octadecylalkohol, Stearylalkohol), ethoxyliert mit 27 EO,
- Octadecan-1-ol (Octadecylalkohol, Stearylalkohol), ethoxyliert mit 28 EO,
- Octadecan-1-ol (Octadecylalkohol, Stearylalkohol), ethoxyliert mit 29 EO,
- Octadecan-1-ol (Octadecylalkohol, Stearylalkohol), ethoxyliert mit 30 EO,
- (9Z)-Octadec-9-en-1-ol (Oleylalkohol), ethoxyliert mit 10 EO,
- (9Z)-Octadec-9-en-1-ol (Oleylalkohol), ethoxyliert mit 11 EO,
- (9Z)-Octadec-9-en-1-ol (Oleylalkohol), ethoxyliert mit 12 EO,
- (9Z)-Octadec-9-en-1-ol (Oleylalkohol), ethoxyliert mit 13 EO,
- (9Z)-Octadec-9-en-1-ol (Oleylalkohol), ethoxyliert mit 14 EO,
- (9Z)-Octadec-9-en-1-ol (Oleylalkohol), ethoxyliert mit 15 EO,
- (9Z)-Octadec-9-en-1-ol (Oleylalkohol), ethoxyliert mit 16 EO,
- (9Z)-Octadec-9-en-1-ol (Oleylalkohol), ethoxyliert mit 17 EO,
- (9Z)-Octadec-9-en-1-ol (Oleylalkohol), ethoxyliert mit 18 EO,
- (9Z)-Octadec-9-en-1-ol (Oleylalkohol), ethoxyliert mit 19 EO,
- (9Z)-Octadec-9-en-1-ol (Oleylalkohol), ethoxyliert mit 20 EO,
- (9Z)-Octadec-9-en-1-ol (Oleylalkohol), ethoxyliert mit 21 EO,
- (9Z)-Octadec-9-en-1-ol (Oleylalkohol), ethoxyliert mit 22 EO,
- (9Z)-Octadec-9-en-1-ol (Oleylalkohol), ethoxyliert mit 23 EO,
- (9Z)-Octadec-9-en-1-ol (Oleylalkohol), ethoxyliert mit 24 EO,
- (9Z)-Octadec-9-en-1-ol (Oleylalkohol), ethoxyliert mit 25 EO,
- (9Z)-Octadec-9-en-1-ol (Oleylalkohol), ethoxyliert mit 26 EO,
- (9Z)-Octadec-9-en-1-ol (Oleylalkohol), ethoxyliert mit 27 EO,
- (9Z)-Octadec-9-en-1-ol (Oleylalkohol), ethoxyliert mit 28 EO,
- (9Z)-Octadec-9-en-1-ol (Oleylalkohol), ethoxyliert mit 29 EO,
- (9Z)-Octadec-9-en-1-ol (Oleylalkohol), ethoxyliert mit 30 EO,
- (9E)-Octadec-9-en-1-ol (Elaidylalkohol), ethoxyliert mit 10 EO,
- (9E)-Octadec-9-en-1-ol (Elaidylalkohol), ethoxyliert mit 11 EO,
- (9E)-Octadec-9-en-1-ol (Elaidylalkohol), ethoxyliert mit 12 EO,
- (9E)-Octadec-9-en-1-ol (Elaidylalkohol), ethoxyliert mit 13 EO,
- (9E)-Octadec-9-en-1-ol (Elaidylalkohol), ethoxyliert mit 14 EO,
- (9E)-Octadec-9-en-1-ol (Elaidylalkohol), ethoxyliert mit 15 EO,
- (9E)-Octadec-9-en-1-ol (Elaidylalkohol), ethoxyliert mit 16 EO,
- (9E)-Octadec-9-en-1-ol (Elaidylalkohol), ethoxyliert mit 17 EO,
- (9E)-Octadec-9-en-1-ol (Elaidylalkohol), ethoxyliert mit 18 EO,
- (9E)-Octadec-9-en-1-ol (Elaidylalkohol), ethoxyliert mit 19 EO,
- (9E)-Octadec-9-en-1-ol (Elaidylalkohol), ethoxyliert mit 20 EO,
- (9E)-Octadec-9-en-1-ol (Elaidylalkohol), ethoxyliert mit 21 EO,
- (9E)-Octadec-9-en-1-ol (Elaidylalkohol), ethoxyliert mit 22 EO,
- (9E)-Octadec-9-en-1-ol (Elaidylalkohol), ethoxyliert mit 23 EO,
- (9E)-Octadec-9-en-1-ol (Elaidylalkohol), ethoxyliert mit 24 EO,
- (9E)-Octadec-9-en-1-ol (Elaidylalkohol), ethoxyliert mit 25 EO,
- (9E)-Octadec-9-en-1-ol (Elaidylalkohol), ethoxyliert mit 26 EO,
- (9E)-Octadec-9-en-1-ol (Elaidylalkohol), ethoxyliert mit 27 EO,
- (9E)-Octadec-9-en-1-ol (Elaidylalkohol), ethoxyliert mit 28 EO,
- (9E)-Octadec-9-en-1-ol (Elaidylalkohol), ethoxyliert mit 29 EO,
- (9E)-Octadec-9-en-1-ol (Elaidylalkohol), ethoxyliert mit 30 EO,
- (9Z,12Z)-Octadeca-9,12-dien-1-ol (Linoleylalkohol), ethoxyliert mit 10 EO,
- (9Z,12Z)-Octadeca-9,12-dien-1-ol (Linoleylalkohol), ethoxyliert mit 11 EO,
- (9Z,12Z)-Octadeca-9,12-dien-1-ol (Linoleylalkohol), ethoxyliert mit 12 EO,
- (9Z,12Z)-Octadeca-9,12-dien-1-ol (Linoleylalkohol), ethoxyliert mit 13 EO,
- (9Z,12Z)-Octadeca-9,12-dien-1-ol (Linoleylalkohol), ethoxyliert mit 14 EO,
- (9Z,12Z)-Octadeca-9,12-dien-1-ol (Linoleylalkohol), ethoxyliert mit 15 EO,
- (9Z,12Z)-Octadeca-9,12-dien-1-ol (Linoleylalkohol), ethoxyliert mit 16 EO,
- (9Z,12Z)-Octadeca-9,12-dien-1-ol (Linoleylalkohol), ethoxyliert mit 17 EO,
- (9Z,12Z)-Octadeca-9,12-dien-1-ol (Linoleylalkohol), ethoxyliert mit 18 EO,
- (9Z,12Z)-Octadeca-9,12-dien-1-ol (Linoleylalkohol), ethoxyliert mit 19 EO,
- (9Z,12Z)-Octadeca-9,12-dien-1-ol (Linoleylalkohol), ethoxyliert mit 20 EO,
- (9Z,12Z)-Octadeca-9,12-dien-1-ol (Linoleylalkohol), ethoxyliert mit 21 EO,
- (9Z,12Z)-Octadeca-9,12-dien-1-ol (Linoleylalkohol), ethoxyliert mit 22 EO,
- (9Z,12Z)-Octadeca-9,12-dien-1-ol (Linoleylalkohol), ethoxyliert mit 23 EO,
- (9Z,12Z)-Octadeca-9,12-dien-1-ol (Linoleylalkohol), ethoxyliert mit 24 EO,
- (9Z,12Z)-Octadeca-9,12-dien-1-ol (Linoleylalkohol), ethoxyliert mit 25 EO,
- (9Z,12Z)-Octadeca-9,12-dien-1-ol (Linoleylalkohol), ethoxyliert mit 26 EO,
- (9Z,12Z)-Octadeca-9,12-dien-1-ol (Linoleylalkohol), ethoxyliert mit 27 EO,
- (9Z,12Z)-Octadeca-9,12-dien-1-ol (Linoleylalkohol), ethoxyliert mit 28 EO,
- (9Z,12Z)-Octadeca-9,12-dien-1-ol (Linoleylalkohol), ethoxyliert mit 29 EO,
- (9Z,12Z)-Octadeca-9,12-dien-1-ol (Linoleylalkohol), ethoxyliert mit 30 EO,
- Eicosan-1-ol (Eicosylalkohol, Arachylalkohol), ethoxyliert mit 10 EO,
- Eicosan-1-ol (Eicosylalkohol, Arachylalkohol), ethoxyliert mit 11 EO,
- Eicosan-1-ol (Eicosylalkohol, Arachylalkohol), ethoxyliert mit 12 EO,
- Eicosan-1-ol (Eicosylalkohol, Arachylalkohol), ethoxyliert mit 13 EO,
- Eicosan-1-ol (Eicosylalkohol, Arachylalkohol), ethoxyliert mit 14 EO,
- Eicosan-1-ol (Eicosylalkohol, Arachylalkohol), ethoxyliert mit 15 EO,
- Eicosan-1-ol (Eicosylalkohol, Arachylalkohol), ethoxyliert mit 16 EO,
- Eicosan-1-ol (Eicosylalkohol, Arachylalkohol), ethoxyliert mit 17 EO,
- Eicosan-1-ol (Eicosylalkohol, Arachylalkohol), ethoxyliert mit 18 EO,
- Eicosan-1-ol (Eicosylalkohol, Arachylalkohol), ethoxyliert mit 19 EO,
- Eicosan-1-ol (Eicosylalkohol, Arachylalkohol), ethoxyliert mit 20 EO,
- Eicosan-1-ol (Eicosylalkohol, Arachylalkohol), ethoxyliert mit 21 EO,
- Eicosan-1-ol (Eicosylalkohol, Arachylalkohol), ethoxyliert mit 22 EO,
- Eicosan-1-ol (Eicosylalkohol, Arachylalkohol), ethoxyliert mit 23 EO,
- Eicosan-1-ol (Eicosylalkohol, Arachylalkohol), ethoxyliert mit 24 EO,
- Eicosan-1-ol (Eicosylalkohol, Arachylalkohol), ethoxyliert mit 25 EO,
- Eicosan-1-ol (Eicosylalkohol, Arachylalkohol), ethoxyliert mit 26 EO,
- Eicosan-1-ol (Eicosylalkohol, Arachylalkohol), ethoxyliert mit 27 EO,
- Eicosan-1-ol (Eicosylalkohol, Arachylalkohol), ethoxyliert mit 28 EO,
- Eicosan-1-ol (Eicosylalkohol, Arachylalkohol), ethoxyliert mit 29 EO,
- Eicosan-1-ol (Eicosylalkohol, Arachylalkohol), ethoxyliert mit 30 EO,
- Docosan-1-ol (Docosylalkohol, Behenylalkohol), ethoxyliert mit 10 EO
- Docosan-1-ol (Docosylalkohol, Behenylalkohol), ethoxyliert mit 11 EO
- Docosan-1-ol (Docosylalkohol, Behenylalkohol), ethoxyliert mit 12 EO
- Docosan-1-ol (Docosylalkohol, Behenylalkohol), ethoxyliert mit 13 EO
- Docosan-1-ol (Docosylalkohol, Behenylalkohol), ethoxyliert mit 14 EO
- Docosan-1-ol (Docosylalkohol, Behenylalkohol), ethoxyliert mit 15 EO
- Docosan-1-ol (Docosylalkohol, Behenylalkohol), ethoxyliert mit 16 EO
- Docosan-1-ol (Docosylalkohol, Behenylalkohol), ethoxyliert mit 17 EO
- Docosan-1-ol (Docosylalkohol, Behenylalkohol), ethoxyliert mit 18 EO
- Docosan-1-ol (Docosylalkohol, Behenylalkohol), ethoxyliert mit 19 EO
- Docosan-1-ol (Docosylalkohol, Behenylalkohol), ethoxyliert mit 20 EO
- Docosan-1-ol (Docosylalkohol, Behenylalkohol), ethoxyliert mit 21 EO
- Docosan-1-ol (Docosylalkohol, Behenylalkohol), ethoxyliert mit 22 EO
- Docosan-1-ol (Docosylalkohol, Behenylalkohol), ethoxyliert mit 23 EO
- Docosan-1-ol (Docosylalkohol, Behenylalkohol), ethoxyliert mit 24 EO
- Docosan-1-ol (Docosylalkohol, Behenylalkohol), ethoxyliert mit 25 EO
- Docosan-1-ol (Docosylalkohol, Behenylalkohol), ethoxyliert mit 26 EO
- Docosan-1-ol (Docosylalkohol, Behenylalkohol), ethoxyliert mit 27 EO
- Docosan-1-ol (Docosylalkohol, Behenylalkohol), ethoxyliert mit 28 EO
- Docosan-1-ol (Docosylalkohol, Behenylalkohol), ethoxyliert mit 29 EO
- Docosan-1-ol (Docosylalkohol, Behenylalkohol), ethoxyliert mit 30 EO,
sowie Mischungen hiervon enthält.

Außerordentlich bevorzugt sind
- Decan-1-ol (Decylalkohol), ethoxyliert mit 10 EO,
- Decan-1-ol (Decylalkohol), ethoxyliert mit 20 EO,
- Decan-1-ol (Decylalkohol), ethoxyliert mit 30 EO,
- Dodecan-1-ol (Dodecylalkohol, Laurylalkohol), ethoxyliert mit 10 EO,
- Dodecan-1-ol (Dodecylalkohol, Laurylalkohol), ethoxyliert mit 20 EO,
- Dodecan-1-ol (Dodecylalkohol, Laurylalkohol), ethoxyliert mit 30 EO,
- Hexadecan-1-ol (Hexadecylalkohol, Cetylalkohol, Palmitylalkohol), ethoxyliert mit 10 EO,
- Hexadecan-1-ol (Hexadecylalkohol, Cetylalkohol, Palmitylalkohol), ethoxyliert mit 12 EO,
- Hexadecan-1-ol (Hexadecylalkohol, Cetylalkohol, Palmitylalkohol), ethoxyliert mit 15 EO,
- Hexadecan-1-ol (Hexadecylalkohol, Cetylalkohol, Palmitylalkohol), ethoxyliert mit 20 EO,
- Hexadecan-1-ol (Hexadecylalkohol, Cetylalkohol, Palmitylalkohol), ethoxyliert mit 25 EO,
- Hexadecan-1-ol (Hexadecylalkohol, Cetylalkohol, Palmitylalkohol), ethoxyliert mit 30 EO,
- Octadecan-1-ol (Octadecylalkohol, Stearylalkohol), ethoxyliert mit 10 EO,
- Octadecan-1-ol (Octadecylalkohol, Stearylalkohol), ethoxyliert mit 12 EO,
- Octadecan-1-ol (Octadecylalkohol, Stearylalkohol), ethoxyliert mit 15 EO,
- Octadecan-1-ol (Octadecylalkohol, Stearylalkohol), ethoxyliert mit 20 EO,
- Octadecan-1-ol (Octadecylalkohol, Stearylalkohol), ethoxyliert mit 25 EO,
- Octadecan-1-ol (Octadecylalkohol, Stearylalkohol), ethoxyliert mit 30 EO,
- Eicosan-1-ol (Eicosylalkohol, Arachylalkohol), ethoxyliert mit 10 EO,
- Eicosan-1-ol (Eicosylalkohol, Arachylalkohol), ethoxyliert mit 12 EO,
- Eicosan-1-ol (Eicosylalkohol, Arachylalkohol), ethoxyliert mit 15 EO,
- Eicosan-1-ol (Eicosylalkohol, Arachylalkohol), ethoxyliert mit 20 EO,
- Eicosan-1-ol (Eicosylalkohol, Arachylalkohol), ethoxyliert mit 25 EO,
- Eicosan-1-ol (Eicosylalkohol, Arachylalkohol), ethoxyliert mit 30 EO,
- Docosan-1-ol (Docosylalkohol, Behenylalkohol), ethoxyliert mit 10 EO
- Docosan-1-ol (Docosylalkohol, Behenylalkohol), ethoxyliert mit 12 EO
- Docosan-1-ol (Docosylalkohol, Behenylalkohol), ethoxyliert mit 15 EO
- Docosan-1-ol (Docosylalkohol, Behenylalkohol), ethoxyliert mit 20 EO
- Docosan-1-ol (Docosylalkohol, Behenylalkohol), ethoxyliert mit 25 EO
- Docosan-1-ol (Docosylalkohol, Behenylalkohol), ethoxyliert mit 30 EO,
sowie Mischungen hiervon.

Als sechsten wesentlichen Formulierungsbestandteil enthalten die erfindungsgemäßen Oxidationszusammensetzungen mindestens einen ethoxylierten Fettalkohol der Formel (IV) 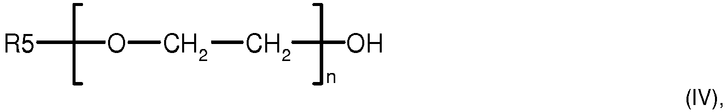 worin
R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und n für eine ganze Zahl von 50 bis 150, bevorzugt für eine ganze Zahl von 80 bis 120, weiter bevorzugt für die Zahl 100 steht,
wobei der mindestens eine ethoxylierte Fettalkohol der Formel (IV) bevorzugt in einer Gesamtmenge von 0,03 - 0,3 Gew.-%, besonders bevorzugt 0,08 bis 0,25 Gew.-%, außerordentlich bevorzugt 0,1 - 0,2 Gew.-%, enthalten ist.

Ganz besonders bevorzugt ist es, wenn als ethoxylierter Fettalkohol der Formel (IV) ein oder mehrere ethoxylierte Fettalkohole aus der Gruppe
- Dodecan-1-ol (Dodecylalkohol, Laurylalkohol), ethoxyliert mit 50 EO,
- Dodecan-1-ol (Dodecylalkohol, Laurylalkohol), ethoxyliert mit 51 EO,
- Dodecan-1-ol (Dodecylalkohol, Laurylalkohol), ethoxyliert mit 52 EO,
- Dodecan-1-ol (Dodecylalkohol, Laurylalkohol), ethoxyliert mit 53 EO,
- Dodecan-1-ol (Dodecylalkohol, Laurylalkohol), ethoxyliert mit 54 EO,
- Dodecan-1-ol (Dodecylalkohol, Laurylalkohol), ethoxyliert mit 55 EO,
- Dodecan-1-ol (Dodecylalkohol, Laurylalkohol), ethoxyliert mit 56 EO,
- Dodecan-1-ol (Dodecylalkohol, Laurylalkohol), ethoxyliert mit 57 EO,
- Dodecan-1-ol (Dodecylalkohol, Laurylalkohol), ethoxyliert mit 58 EO,
- Dodecan-1-ol (Dodecylalkohol, Laurylalkohol), ethoxyliert mit 59 EO,
- Dodecan-1-ol (Dodecylalkohol, Laurylalkohol), ethoxyliert mit 60 EO,
- Dodecan-1-ol (Dodecylalkohol, Laurylalkohol), ethoxyliert mit 80 EO,
- Dodecan-1-ol (Dodecylalkohol, Laurylalkohol), ethoxyliert mit 81 EO,
- Dodecan-1-ol (Dodecylalkohol, Laurylalkohol), ethoxyliert mit 82 EO,
- Dodecan-1-ol (Dodecylalkohol, Laurylalkohol), ethoxyliert mit 83 EO,
- Dodecan-1-ol (Dodecylalkohol, Laurylalkohol), ethoxyliert mit 84 EO,
- Dodecan-1-ol (Dodecylalkohol, Laurylalkohol), ethoxyliert mit 85 EO,
- Dodecan-1-ol (Dodecylalkohol, Laurylalkohol), ethoxyliert mit 86 EO,
- Dodecan-1-ol (Dodecylalkohol, Laurylalkohol), ethoxyliert mit 87 EO,
- Dodecan-1-ol (Dodecylalkohol, Laurylalkohol), ethoxyliert mit 88 EO,
- Dodecan-1-ol (Dodecylalkohol, Laurylalkohol), ethoxyliert mit 89 EO,
- Dodecan-1-ol (Dodecylalkohol, Laurylalkohol), ethoxyliert mit 90 EO,
- Dodecan-1-ol (Dodecylalkohol, Laurylalkohol), ethoxyliert mit 91 EO,
- Dodecan-1-ol (Dodecylalkohol, Laurylalkohol), ethoxyliert mit 92 EO,
- Dodecan-1-ol (Dodecylalkohol, Laurylalkohol), ethoxyliert mit 93 EO,
- Dodecan-1-ol (Dodecylalkohol, Laurylalkohol), ethoxyliert mit 94 EO,
- Dodecan-1-ol (Dodecylalkohol, Laurylalkohol), ethoxyliert mit 95 EO,
- Dodecan-1-ol (Dodecylalkohol, Laurylalkohol), ethoxyliert mit 96 EO,
- Dodecan-1-ol (Dodecylalkohol, Laurylalkohol), ethoxyliert mit 97 EO,
- Dodecan-1-ol (Dodecylalkohol, Laurylalkohol), ethoxyliert mit 98 EO,
- Dodecan-1-ol (Dodecylalkohol, Laurylalkohol), ethoxyliert mit 99 EO,
- Dodecan-1-ol (Dodecylalkohol, Laurylalkohol), ethoxyliert mit 100 EO,
- Dodecan-1-ol (Dodecylalkohol, Laurylalkohol), ethoxyliert mit 101 EO,
- Dodecan-1-ol (Dodecylalkohol, Laurylalkohol), ethoxyliert mit 102 EO,
- Dodecan-1-ol (Dodecylalkohol, Laurylalkohol), ethoxyliert mit 103 EO,
- Dodecan-1-ol (Dodecylalkohol, Laurylalkohol), ethoxyliert mit 104 EO,
- Dodecan-1-ol (Dodecylalkohol, Laurylalkohol), ethoxyliert mit 105 EO,
- Dodecan-1-ol (Dodecylalkohol, Laurylalkohol), ethoxyliert mit 106 EO,
- Dodecan-1-ol (Dodecylalkohol, Laurylalkohol), ethoxyliert mit 107 EO,
- Dodecan-1-ol (Dodecylalkohol, Laurylalkohol), ethoxyliert mit 108 EO,
- Dodecan-1-ol (Dodecylalkohol, Laurylalkohol), ethoxyliert mit 109 EO,
- Dodecan-1-ol (Dodecylalkohol, Laurylalkohol), ethoxyliert mit 110 EO,
- Dodecan-1-ol (Dodecylalkohol, Laurylalkohol), ethoxyliert mit 111 EO,
- Dodecan-1-ol (Dodecylalkohol, Laurylalkohol), ethoxyliert mit 112 EO,
- Dodecan-1-ol (Dodecylalkohol, Laurylalkohol), ethoxyliert mit 113 EO,
- Dodecan-1-ol (Dodecylalkohol, Laurylalkohol), ethoxyliert mit 114 EO,
- Dodecan-1-ol (Dodecylalkohol, Laurylalkohol), ethoxyliert mit 115 EO,
- Dodecan-1-ol (Dodecylalkohol, Laurylalkohol), ethoxyliert mit 116 EO,
- Dodecan-1-ol (Dodecylalkohol, Laurylalkohol), ethoxyliert mit 117 EO,
- Dodecan-1-ol (Dodecylalkohol, Laurylalkohol), ethoxyliert mit 118 EO,
- Dodecan-1-ol (Dodecylalkohol, Laurylalkohol), ethoxyliert mit 119 EO,
- Dodecan-1-ol (Dodecylalkohol, Laurylalkohol), ethoxyliert mit 120 EO,
- Tetradecan-1-ol, (Tetradecylalkohol, Myristylalkohol), ethoxyliert mit 50 EO,
- Tetradecan-1-ol, (Tetradecylalkohol, Myristylalkohol), ethoxyliert mit 51 EO,
- Tetradecan-1-ol, (Tetradecylalkohol, Myristylalkohol), ethoxyliert mit 52 EO,
- Tetradecan-1-ol, (Tetradecylalkohol, Myristylalkohol), ethoxyliert mit 53 EO,
- Tetradecan-1-ol, (Tetradecylalkohol, Myristylalkohol), ethoxyliert mit 54 EO,
- Tetradecan-1-ol, (Tetradecylalkohol, Myristylalkohol), ethoxyliert mit 55 EO,
- Tetradecan-1-ol, (Tetradecylalkohol, Myristylalkohol), ethoxyliert mit 56 EO,
- Tetradecan-1-ol, (Tetradecylalkohol, Myristylalkohol), ethoxyliert mit 57 EO,
- Tetradecan-1-ol, (Tetradecylalkohol, Myristylalkohol), ethoxyliert mit 58 EO,
- Tetradecan-1-ol, (Tetradecylalkohol, Myristylalkohol), ethoxyliert mit 59 EO,
- Tetradecan-1-ol, (Tetradecylalkohol, Myristylalkohol), ethoxyliert mit 60 EO,
- Tetradecan-1-ol, (Tetradecylalkohol, Myristylalkohol), ethoxyliert mit 80 EO,
- Tetradecan-1-ol, (Tetradecylalkohol, Myristylalkohol), ethoxyliert mit 81 EO,
- Tetradecan-1-ol, (Tetradecylalkohol, Myristylalkohol), ethoxyliert mit 82 EO,
- Tetradecan-1-ol, (Tetradecylalkohol, Myristylalkohol), ethoxyliert mit 83 EO,
- Tetradecan-1-ol, (Tetradecylalkohol, Myristylalkohol), ethoxyliert mit 84 EO,
- Tetradecan-1-ol, (Tetradecylalkohol, Myristylalkohol), ethoxyliert mit 85 EO,
- Tetradecan-1-ol, (Tetradecylalkohol, Myristylalkohol), ethoxyliert mit 86 EO,
- Tetradecan-1-ol, (Tetradecylalkohol, Myristylalkohol), ethoxyliert mit 87 EO,
- Tetradecan-1-ol, (Tetradecylalkohol, Myristylalkohol), ethoxyliert mit 88 EO,
- Tetradecan-1-ol, (Tetradecylalkohol, Myristylalkohol), ethoxyliert mit 89 EO,
- Tetradecan-1-ol, (Tetradecylalkohol, Myristylalkohol), ethoxyliert mit 90 EO,
- Tetradecan-1-ol, (Tetradecylalkohol, Myristylalkohol), ethoxyliert mit 91 EO,
- Tetradecan-1-ol, (Tetradecylalkohol, Myristylalkohol), ethoxyliert mit 92 EO,
- Tetradecan-1-ol, (Tetradecylalkohol, Myristylalkohol), ethoxyliert mit 93 EO,
- Tetradecan-1-ol, (Tetradecylalkohol, Myristylalkohol), ethoxyliert mit 94 EO,
- Tetradecan-1-ol, (Tetradecylalkohol, Myristylalkohol), ethoxyliert mit 95 EO,
- Tetradecan-1-ol, (Tetradecylalkohol, Myristylalkohol), ethoxyliert mit 96 EO,
- Tetradecan-1-ol, (Tetradecylalkohol, Myristylalkohol), ethoxyliert mit 97 EO,
- Tetradecan-1-ol, (Tetradecylalkohol, Myristylalkohol), ethoxyliert mit 98 EO,
- Tetradecan-1-ol, (Tetradecylalkohol, Myristylalkohol), ethoxyliert mit 99 EO,
- Tetradecan-1-ol, (Tetradecylalkohol, Myristylalkohol), ethoxyliert mit 100 EO,
- Tetradecan-1-ol, (Tetradecylalkohol, Myristylalkohol), ethoxyliert mit 101 EO,
- Tetradecan-1-ol, (Tetradecylalkohol, Myristylalkohol), ethoxyliert mit 102 EO,
- Tetradecan-1-ol, (Tetradecylalkohol, Myristylalkohol), ethoxyliert mit 103 EO,
- Tetradecan-1-ol, (Tetradecylalkohol, Myristylalkohol), ethoxyliert mit 104 EO,
- Tetradecan-1-ol, (Tetradecylalkohol, Myristylalkohol), ethoxyliert mit 105 EO,
- Tetradecan-1-ol, (Tetradecylalkohol, Myristylalkohol), ethoxyliert mit 106 EO,
- Tetradecan-1-ol, (Tetradecylalkohol, Myristylalkohol), ethoxyliert mit 107 EO,
- Tetradecan-1-ol, (Tetradecylalkohol, Myristylalkohol), ethoxyliert mit 108 EO,
- Tetradecan-1-ol, (Tetradecylalkohol, Myristylalkohol), ethoxyliert mit 109 EO,
- Tetradecan-1-ol, (Tetradecylalkohol, Myristylalkohol), ethoxyliert mit 110 EO,
- Tetradecan-1-ol, (Tetradecylalkohol, Myristylalkohol), ethoxyliert mit 111 EO,
- Tetradecan-1-ol, (Tetradecylalkohol, Myristylalkohol), ethoxyliert mit 112 EO,
- Tetradecan-1-ol, (Tetradecylalkohol, Myristylalkohol), ethoxyliert mit 113 EO,
- Tetradecan-1-ol, (Tetradecylalkohol, Myristylalkohol), ethoxyliert mit 114 EO,
- Tetradecan-1-ol, (Tetradecylalkohol, Myristylalkohol), ethoxyliert mit 115 EO,
- Tetradecan-1-ol, (Tetradecylalkohol, Myristylalkohol), ethoxyliert mit 116 EO,
- Tetradecan-1-ol, (Tetradecylalkohol, Myristylalkohol), ethoxyliert mit 117 EO,
- Tetradecan-1-ol, (Tetradecylalkohol, Myristylalkohol), ethoxyliert mit 118 EO,
- Tetradecan-1-ol, (Tetradecylalkohol, Myristylalkohol), ethoxyliert mit 119 EO,
- Tetradecan-1-ol, (Tetradecylalkohol, Myristylalkohol), ethoxyliert mit 120 EO,
- Hexadecan-1-ol (Hexadecylalkohol, Cetylalkohol, Palmitylalkohol), ethoxyliert mit 50 EO,
- Hexadecan-1-ol (Hexadecylalkohol, Cetylalkohol, Palmitylalkohol), ethoxyliert mit 51 EO,
- Hexadecan-1-ol (Hexadecylalkohol, Cetylalkohol, Palmitylalkohol), ethoxyliert mit 52 EO,
- Hexadecan-1-ol (Hexadecylalkohol, Cetylalkohol, Palmitylalkohol), ethoxyliert mit 53 EO,
- Hexadecan-1-ol (Hexadecylalkohol, Cetylalkohol, Palmitylalkohol), ethoxyliert mit 54 EO,
- Hexadecan-1-ol (Hexadecylalkohol, Cetylalkohol, Palmitylalkohol), ethoxyliert mit 55 EO,
- Hexadecan-1-ol (Hexadecylalkohol, Cetylalkohol, Palmitylalkohol), ethoxyliert mit 56 EO,
- Hexadecan-1-ol (Hexadecylalkohol, Cetylalkohol, Palmitylalkohol), ethoxyliert mit 57 EO,
- Hexadecan-1-ol (Hexadecylalkohol, Cetylalkohol, Palmitylalkohol), ethoxyliert mit 58 EO,
- Hexadecan-1-ol (Hexadecylalkohol, Cetylalkohol, Palmitylalkohol), ethoxyliert mit 59 EO,
- Hexadecan-1-ol (Hexadecylalkohol, Cetylalkohol, Palmitylalkohol), ethoxyliert mit 60 EO,
- Hexadecan-1-ol (Hexadecylalkohol, Cetylalkohol, Palmitylalkohol), ethoxyliert mit 80 EO,
- Hexadecan-1-ol (Hexadecylalkohol, Cetylalkohol, Palmitylalkohol), ethoxyliert mit 81 EO,
- Hexadecan-1-ol (Hexadecylalkohol, Cetylalkohol, Palmitylalkohol), ethoxyliert mit 82 EO,
- Hexadecan-1-ol (Hexadecylalkohol, Cetylalkohol, Palmitylalkohol), ethoxyliert mit 83 EO,
- Hexadecan-1-ol (Hexadecylalkohol, Cetylalkohol, Palmitylalkohol), ethoxyliert mit 84 EO,
- Hexadecan-1-ol (Hexadecylalkohol, Cetylalkohol, Palmitylalkohol), ethoxyliert mit 85 EO,
- Hexadecan-1-ol (Hexadecylalkohol, Cetylalkohol, Palmitylalkohol), ethoxyliert mit 86 EO,
- Hexadecan-1-ol (Hexadecylalkohol, Cetylalkohol, Palmitylalkohol), ethoxyliert mit 87 EO,
- Hexadecan-1-ol (Hexadecylalkohol, Cetylalkohol, Palmitylalkohol), ethoxyliert mit 88 EO,
- Hexadecan-1-ol (Hexadecylalkohol, Cetylalkohol, Palmitylalkohol), ethoxyliert mit 89 EO,
- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Hexadecan-1-ol (Hexadecylalkohol, Cetylalkohol, Palmitylalkohol), ethoxyliert mit 111 EO,
- Hexadecan-1-ol (Hexadecylalkohol, Cetylalkohol, Palmitylalkohol), ethoxyliert mit 112 EO,
- Hexadecan-1-ol (Hexadecylalkohol, Cetylalkohol, Palmitylalkohol), ethoxyliert mit 113 EO,
- Hexadecan-1-ol (Hexadecylalkohol, Cetylalkohol, Palmitylalkohol), ethoxyliert mit 114 EO,
- Hexadecan-1-ol (Hexadecylalkohol, Cetylalkohol, Palmitylalkohol), ethoxyliert mit 115 EO,
- Hexadecan-1-ol (Hexadecylalkohol, Cetylalkohol, Palmitylalkohol), ethoxyliert mit 116 EO,
- Hexadecan-1-ol (Hexadecylalkohol, Cetylalkohol, Palmitylalkohol), ethoxyliert mit 117 EO,
- Hexadecan-1-ol (Hexadecylalkohol, Cetylalkohol, Palmitylalkohol), ethoxyliert mit 118 EO,
- Hexadecan-1-ol (Hexadecylalkohol, Cetylalkohol, Palmitylalkohol), ethoxyliert mit 119 EO,
- Hexadecan-1-ol (Hexadecylalkohol, Cetylalkohol, Palmitylalkohol), ethoxyliert mit 120 EO,
- Octadecan-1-ol (Octadecylalkohol, Stearylalkohol), ethoxyliert mit 50 EO,
- Octadecan-1-ol (Octadecylalkohol, Stearylalkohol), ethoxyliert mit 51 EO,
- Octadecan-1-ol (Octadecylalkohol, Stearylalkohol), ethoxyliert mit 52 EO,
- Octadecan-1-ol (Octadecylalkohol, Stearylalkohol), ethoxyliert mit 53 EO,
- Octadecan-1-ol (Octadecylalkohol, Stearylalkohol), ethoxyliert mit 54 EO,
- Octadecan-1-ol (Octadecylalkohol, Stearylalkohol), ethoxyliert mit 55 EO,
- Octadecan-1-ol (Octadecylalkohol, Stearylalkohol), ethoxyliert mit 56 EO,
- Octadecan-1-ol (Octadecylalkohol, Stearylalkohol), ethoxyliert mit 57 EO,
- Octadecan-1-ol (Octadecylalkohol, Stearylalkohol), ethoxyliert mit 58 EO,
- Octadecan-1-ol (Octadecylalkohol, Stearylalkohol), ethoxyliert mit 59 EO,
- Octadecan-1-ol (Octadecylalkohol, Stearylalkohol), ethoxyliert mit 60 EO,
- Octadecan-1-ol (Octadecylalkohol, Stearylalkohol), ethoxyliert mit 80 EO,
- Octadecan-1-ol (Octadecylalkohol, Stearylalkohol), ethoxyliert mit 81 EO,
- Octadecan-1-ol (Octadecylalkohol, Stearylalkohol), ethoxyliert mit 82 EO,
- Octadecan-1-ol (Octadecylalkohol, Stearylalkohol), ethoxyliert mit 83 EO,
- Octadecan-1-ol (Octadecylalkohol, Stearylalkohol), ethoxyliert mit 84 EO,
- Octadecan-1-ol (Octadecylalkohol, Stearylalkohol), ethoxyliert mit 85 EO,
- Octadecan-1-ol (Octadecylalkohol, Stearylalkohol), ethoxyliert mit 86 EO,
- Octadecan-1-ol (Octadecylalkohol, Stearylalkohol), ethoxyliert mit 87 EO,
- Octadecan-1-ol (Octadecylalkohol, Stearylalkohol), ethoxyliert mit 88 EO,
- Octadecan-1-ol (Octadecylalkohol, Stearylalkohol), ethoxyliert mit 89 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,
- Octadecan-1-ol (Octadecylalkohol, Stearylalkohol), ethoxyliert mit 111 EO,
- Octadecan-1-ol (Octadecylalkohol, Stearylalkohol), ethoxyliert mit 112 EO,
- Octadecan-1-ol (Octadecylalkohol, Stearylalkohol), ethoxyliert mit 113 EO,
- Octadecan-1-ol (Octadecylalkohol, Stearylalkohol), ethoxyliert mit 114 EO,
- Octadecan-1-ol (Octadecylalkohol, Stearylalkohol), ethoxyliert mit 115 EO,
- Octadecan-1-ol (Octadecylalkohol, Stearylalkohol), ethoxyliert mit 116 EO,
- Octadecan-1-ol (Octadecylalkohol, Stearylalkohol), ethoxyliert mit 117 EO,
- Octadecan-1-ol (Octadecylalkohol, Stearylalkohol), ethoxyliert mit 118 EO,
- Octadecan-1-ol (Octadecylalkohol, Stearylalkohol), ethoxyliert mit 119 EO,
- Octadecan-1-ol (Octadecylalkohol, Stearylalkohol), ethoxyliert mit 120 EO,
- (9Z)-Octadec-9-en-1-ol (Oleylalkohol), ethoxyliert mit 50 EO,
- (9Z)-Octadec-9-en-1-ol (Oleylalkohol), ethoxyliert mit 51 EO,
- (9Z)-Octadec-9-en-1-ol (Oleylalkohol), ethoxyliert mit 52 EO,
- (9Z)-Octadec-9-en-1-ol (Oleylalkohol), ethoxyliert mit 53 EO,
- (9Z)-Octadec-9-en-1-ol (Oleylalkohol), ethoxyliert mit 54 EO,
- (9Z)-Octadec-9-en-1-ol (Oleylalkohol), ethoxyliert mit 55 EO,
- (9Z)-Octadec-9-en-1-ol (Oleylalkohol), ethoxyliert mit 56 EO,
- (9Z)-Octadec-9-en-1-ol (Oleylalkohol), ethoxyliert mit 57 EO,
- (9Z)-Octadec-9-en-1-ol (Oleylalkohol), ethoxyliert mit 58 EO,
- (9Z)-Octadec-9-en-1-ol (Oleylalkohol), ethoxyliert mit 59 EO,
- (9Z)-Octadec-9-en-1-ol (Oleylalkohol), ethoxyliert mit 60 EO,
- (9Z)-Octadec-9-en-1-ol (Oleylalkohol), ethoxyliert mit 80 EO,
- (9Z)-Octadec-9-en-1-ol (Oleylalkohol), ethoxyliert mit 81 EO,
- (9Z)-Octadec-9-en-1-ol (Oleylalkohol), ethoxyliert mit 82 EO,
- (9Z)-Octadec-9-en-1-ol (Oleylalkohol), ethoxyliert mit 83 EO,
- (9Z)-Octadec-9-en-1-ol (Oleylalkohol), ethoxyliert mit 84 EO,
- (9Z)-Octadec-9-en-1-ol (Oleylalkohol), ethoxyliert mit 85 EO,
- (9Z)-Octadec-9-en-1-ol (Oleylalkohol), ethoxyliert mit 86 EO,
- (9Z)-Octadec-9-en-1-ol (Oleylalkohol), ethoxyliert mit 87 EO,
- (9Z)-Octadec-9-en-1-ol (Oleylalkohol), ethoxyliert mit 88 EO,
- (9Z)-Octadec-9-en-1-ol (Oleylalkohol), ethoxyliert mit 89 EO,
- (9Z)-Octadec-9-en-1-ol (Oleylalkohol), ethoxyliert mit 90 EO,
- (9Z)-Octadec-9-en-1-ol (Oleylalkohol), ethoxyliert mit 91 EO,
- (9Z)-Octadec-9-en-1-ol (Oleylalkohol), ethoxyliert mit 92 EO,
- (9Z)-Octadec-9-en-1-ol (Oleylalkohol), ethoxyliert mit 93 EO,
- (9Z)-Octadec-9-en-1-ol (Oleylalkohol), ethoxyliert mit 94 EO,
- (9Z)-Octadec-9-en-1-ol (Oleylalkohol), ethoxyliert mit 95 EO,
- (9Z)-Octadec-9-en-1-ol (Oleylalkohol), ethoxyliert mit 96 EO,
- (9Z)-Octadec-9-en-1-ol (Oleylalkohol), ethoxyliert mit 97 EO,
- (9Z)-Octadec-9-en-1-ol (Oleylalkohol), ethoxyliert mit 98 EO,
- (9Z)-Octadec-9-en-1-ol (Oleylalkohol), ethoxyliert mit 99 EO,
- (9Z)-Octadec-9-en-1-ol (Oleylalkohol), ethoxyliert mit 100 EO,
- (9Z)-Octadec-9-en-1-ol (Oleylalkohol), ethoxyliert mit 101 EO,
- (9Z)-Octadec-9-en-1-ol (Oleylalkohol), ethoxyliert mit 102 EO,
- (9Z)-Octadec-9-en-1-ol (Oleylalkohol), ethoxyliert mit 103 EO,
- (9Z)-Octadec-9-en-1-ol (Oleylalkohol), ethoxyliert mit 104 EO,
- (9Z)-Octadec-9-en-1-ol (Oleylalkohol), ethoxyliert mit 105 EO,
- (9Z)-Octadec-9-en-1-ol (Oleylalkohol), ethoxyliert mit 106 EO,
- (9Z)-Octadec-9-en-1-ol (Oleylalkohol), ethoxyliert mit 107 EO,
- (9Z)-Octadec-9-en-1-ol (Oleylalkohol), ethoxyliert mit 108 EO,
- (9Z)-Octadec-9-en-1-ol (Oleylalkohol), ethoxyliert mit 109 EO,
- (9Z)-Octadec-9-en-1-ol (Oleylalkohol), ethoxyliert mit 110 EO,
- (9Z)-Octadec-9-en-1-ol (Oleylalkohol), ethoxyliert mit 111 EO,
- (9Z)-Octadec-9-en-1-ol (Oleylalkohol), ethoxyliert mit 112 EO,
- (9Z)-Octadec-9-en-1-ol (Oleylalkohol), ethoxyliert mit 113 EO,
- (9Z)-Octadec-9-en-1-ol (Oleylalkohol), ethoxyliert mit 114 EO,
- (9Z)-Octadec-9-en-1-ol (Oleylalkohol), ethoxyliert mit 115 EO,
- (9Z)-Octadec-9-en-1-ol (Oleylalkohol), ethoxyliert mit 116 EO,
- (9Z)-Octadec-9-en-1-ol (Oleylalkohol), ethoxyliert mit 117 EO,
- (9Z)-Octadec-9-en-1-ol (Oleylalkohol), ethoxyliert mit 118 EO,
- (9Z)-Octadec-9-en-1-ol (Oleylalkohol), ethoxyliert mit 119 EO,
- (9Z)-Octadec-9-en-1-ol (Oleylalkohol), ethoxyliert mit 120 EO,
- (9E)-Octadec-9-en-1-ol (Elaidylalkohol), ethoxyliert mit 50 EO,
- (9E)-Octadec-9-en-1-ol (Elaidylalkohol), ethoxyliert mit 51 EO,
- (9E)-Octadec-9-en-1-ol (Elaidylalkohol), ethoxyliert mit 52 EO,
- (9E)-Octadec-9-en-1-ol (Elaidylalkohol), ethoxyliert mit 53 EO,
- (9E)-Octadec-9-en-1-ol (Elaidylalkohol), ethoxyliert mit 54 EO,
- (9E)-Octadec-9-en-1-ol (Elaidylalkohol), ethoxyliert mit 55 EO,
- (9E)-Octadec-9-en-1-ol (Elaidylalkohol), ethoxyliert mit 56 EO,
- (9E)-Octadec-9-en-1-ol (Elaidylalkohol), ethoxyliert mit 58 EO,
- (9E)-Octadec-9-en-1-ol (Elaidylalkohol), ethoxyliert mit 55 EO,
- (9E)-Octadec-9-en-1-ol (Elaidylalkohol), ethoxyliert mit 59 EO,
- (9E)-Octadec-9-en-1-ol (Elaidylalkohol), ethoxyliert mit 60 EO,
- (9E)-Octadec-9-en-1-ol (Elaidylalkohol), ethoxyliert mit 80 EO,
- (9E)-Octadec-9-en-1-ol (Elaidylalkohol), ethoxyliert mit 81 EO,
- (9E)-Octadec-9-en-1-ol (Elaidylalkohol), ethoxyliert mit 82 EO,
- (9E)-Octadec-9-en-1-ol (Elaidylalkohol), ethoxyliert mit 83 EO,
- (9E)-Octadec-9-en-1-ol (Elaidylalkohol), ethoxyliert mit 84 EO,
- (9E)-Octadec-9-en-1-ol (Elaidylalkohol), ethoxyliert mit 85 EO,
- (9E)-Octadec-9-en-1-ol (Elaidylalkohol), ethoxyliert mit 86 EO,
- (9E)-Octadec-9-en-1-ol (Elaidylalkohol), ethoxyliert mit 87 EO,
- (9E)-Octadec-9-en-1-ol (Elaidylalkohol), ethoxyliert mit 88 EO,
- (9E)-Octadec-9-en-1-ol (Elaidylalkohol), ethoxyliert mit 89 EO,
- (9E)-Octadec-9-en-1-ol (Elaidylalkohol), ethoxyliert mit 90 EO,
- (9E)-Octadec-9-en-1-ol (Elaidylalkohol), ethoxyliert mit 91 EO,
- (9E)-Octadec-9-en-1-ol (Elaidylalkohol), ethoxyliert mit 92 EO,
- (9E)-Octadec-9-en-1-ol (Elaidylalkohol), ethoxyliert mit 93 EO,
- (9E)-Octadec-9-en-1-ol (Elaidylalkohol), ethoxyliert mit 94 EO,
- (9E)-Octadec-9-en-1-ol (Elaidylalkohol), ethoxyliert mit 95 EO,
- (9E)-Octadec-9-en-1-ol (Elaidylalkohol), ethoxyliert mit 96 EO,
- (9E)-Octadec-9-en-1-ol (Elaidylalkohol), ethoxyliert mit 97 EO,
- (9E)-Octadec-9-en-1-ol (Elaidylalkohol), ethoxyliert mit 98 EO,
- (9E)-Octadec-9-en-1-ol (Elaidylalkohol), ethoxyliert mit 99 EO,
- (9E)-Octadec-9-en-1-ol (Elaidylalkohol), ethoxyliert mit 100 EO,
- (9E)-Octadec-9-en-1-ol (Elaidylalkohol), ethoxyliert mit 101 EO,
- (9E)-Octadec-9-en-1-ol (Elaidylalkohol), ethoxyliert mit 102 EO,
- (9E)-Octadec-9-en-1-ol (Elaidylalkohol), ethoxyliert mit 103 EO,
- (9E)-Octadec-9-en-1-ol (Elaidylalkohol), ethoxyliert mit 104 EO,
- (9E)-Octadec-9-en-1-ol (Elaidylalkohol), ethoxyliert mit 105 EO,
- (9E)-Octadec-9-en-1-ol (Elaidylalkohol), ethoxyliert mit 106 EO,
- (9E)-Octadec-9-en-1-ol (Elaidylalkohol), ethoxyliert mit 107 EO,
- (9E)-Octadec-9-en-1-ol (Elaidylalkohol), ethoxyliert mit 108 EO,
- (9E)-Octadec-9-en-1-ol (Elaidylalkohol), ethoxyliert mit 109 EO,
- (9E)-Octadec-9-en-1-ol (Elaidylalkohol), ethoxyliert mit 110 EO,
- (9E)-Octadec-9-en-1-ol (Elaidylalkohol), ethoxyliert mit 111 EO,
- (9E)-Octadec-9-en-1-ol (Elaidylalkohol), ethoxyliert mit 112 EO,
- (9E)-Octadec-9-en-1-ol (Elaidylalkohol), ethoxyliert mit 113 EO,
- (9E)-Octadec-9-en-1-ol (Elaidylalkohol), ethoxyliert mit 114 EO,
- (9E)-Octadec-9-en-1-ol (Elaidylalkohol), ethoxyliert mit 115 EO,
- (9E)-Octadec-9-en-1-ol (Elaidylalkohol), ethoxyliert mit 116 EO,
- (9E)-Octadec-9-en-1-ol (Elaidylalkohol), ethoxyliert mit 117 EO,
- (9E)-Octadec-9-en-1-ol (Elaidylalkohol), ethoxyliert mit 118 EO,
- (9E)-Octadec-9-en-1-ol (Elaidylalkohol), ethoxyliert mit 119 EO,
- (9E)-Octadec-9-en-1-ol (Elaidylalkohol), ethoxyliert mit 120 EO,
- (9Z,12Z)-Octadeca-9,12-dien-1-ol (Linoleylalkohol), ethoxyliert mit 50 EO,
- (9Z,12Z)-Octadeca-9,12-dien-1-ol (Linoleylalkohol), ethoxyliert mit 51 EO,
- (9Z,12Z)-Octadeca-9,12-dien-1-ol (Linoleylalkohol), ethoxyliert mit 52 EO,
- (9Z,12Z)-Octadeca-9,12-dien-1-ol (Linoleylalkohol), ethoxyliert mit 53 EO,
- (9Z,12Z)-Octadeca-9,12-dien-1-ol (Linoleylalkohol), ethoxyliert mit 54 EO,
- (9Z,12Z)-Octadeca-9,12-dien-1-ol (Linoleylalkohol), ethoxyliert mit 55 EO,
- (9Z,12Z)-Octadeca-9,12-dien-1-ol (Linoleylalkohol), ethoxyliert mit 56 EO,
- (9Z,12Z)-Octadeca-9,12-dien-1-ol (Linoleylalkohol), ethoxyliert mit 57 EO,
- (9Z,12Z)-Octadeca-9,12-dien-1-ol (Linoleylalkohol), ethoxyliert mit 58 EO,
- (9Z,12Z)-Octadeca-9,12-dien-1-ol (Linoleylalkohol), ethoxyliert mit 59 EO,
- (9Z,12Z)-Octadeca-9,12-dien-1-ol (Linoleylalkohol), ethoxyliert mit 60 EO,
- (9Z,12Z)-Octadeca-9,12-dien-1-ol (Linoleylalkohol), ethoxyliert mit 80 EO,
- (9Z,12Z)-Octadeca-9,12-dien-1-ol (Linoleylalkohol), ethoxyliert mit 81 EO,
- (9Z,12Z)-Octadeca-9,12-dien-1-ol (Linoleylalkohol), ethoxyliert mit 82 EO,
- (9Z,12Z)-Octadeca-9,12-dien-1-ol (Linoleylalkohol), ethoxyliert mit 83 EO,
- (9Z,12Z)-Octadeca-9,12-dien-1-ol (Linoleylalkohol), ethoxyliert mit 84 EO,
- (9Z,12Z)-Octadeca-9,12-dien-1-ol (Linoleylalkohol), ethoxyliert mit 85 EO,
- (9Z,12Z)-Octadeca-9,12-dien-1-ol (Linoleylalkohol), ethoxyliert mit 86 EO,
- (9Z,12Z)-Octadeca-9,12-dien-1-ol (Linoleylalkohol), ethoxyliert mit 87 EO,
- (9Z,12Z)-Octadeca-9,12-dien-1-ol (Linoleylalkohol), ethoxyliert mit 88 EO,
- (9Z,12Z)-Octadeca-9,12-dien-1-ol (Linoleylalkohol), ethoxyliert mit 89 EO,
- (9Z,12Z)-Octadeca-9,12-dien-1-ol (Linoleylalkohol), ethoxyliert mit 90 EO,
- (9Z,12Z)-Octadeca-9,12-dien-1-ol (Linoleylalkohol), ethoxyliert mit 91 EO,
- (9Z,12Z)-Octadeca-9,12-dien-1-ol (Linoleylalkohol), ethoxyliert mit 92 EO,
- (9Z,12Z)-Octadeca-9,12-dien-1-ol (Linoleylalkohol), ethoxyliert mit 93 EO,
- (9Z,12Z)-Octadeca-9,12-dien-1-ol (Linoleylalkohol), ethoxyliert mit 94 EO,
- (9Z,12Z)-Octadeca-9,12-dien-1-ol (Linoleylalkohol), ethoxyliert mit 95 EO,
- (9Z,12Z)-Octadeca-9,12-dien-1-ol (Linoleylalkohol), ethoxyliert mit 96 EO,
- (9Z,12Z)-Octadeca-9,12-dien-1-ol (Linoleylalkohol), ethoxyliert mit 97 EO,
- (9Z,12Z)-Octadeca-9,12-dien-1-ol (Linoleylalkohol), ethoxyliert mit 98 EO,
- (9Z,12Z)-Octadeca-9,12-dien-1-ol (Linoleylalkohol), ethoxyliert mit 99 EO,
- (9Z,12Z)-Octadeca-9,12-dien-1-ol (Linoleylalkohol), ethoxyliert mit 100 EO,
- (9Z,12Z)-Octadeca-9,12-dien-1-ol (Linoleylalkohol), ethoxyliert mit 101 EO,
- (9Z,12Z)-Octadeca-9,12-dien-1-ol (Linoleylalkohol), ethoxyliert mit 102 EO,
- (9Z,12Z)-Octadeca-9,12-dien-1-ol (Linoleylalkohol), ethoxyliert mit 103 EO,
- (9Z,12Z)-Octadeca-9,12-dien-1-ol (Linoleylalkohol), ethoxyliert mit 104 EO,
- (9Z,12Z)-Octadeca-9,12-dien-1-ol (Linoleylalkohol), ethoxyliert mit 105 EO,
- (9Z,12Z)-Octadeca-9,12-dien-1-ol (Linoleylalkohol), ethoxyliert mit 106 EO,
- (9Z,12Z)-Octadeca-9,12-dien-1-ol (Linoleylalkohol), ethoxyliert mit 107 EO,
- (9Z,12Z)-Octadeca-9,12-dien-1-ol (Linoleylalkohol), ethoxyliert mit 108 EO,
- (9Z,12Z)-Octadeca-9,12-dien-1-ol (Linoleylalkohol), ethoxyliert mit 109 EO,
- (9Z,12Z)-Octadeca-9,12-dien-1-ol (Linoleylalkohol), ethoxyliert mit 110 EO,
- (9Z,12Z)-Octadeca-9,12-dien-1-ol (Linoleylalkohol), ethoxyliert mit 111 EO,
- (9Z,12Z)-Octadeca-9,12-dien-1-ol (Linoleylalkohol), ethoxyliert mit 112 EO,
- (9Z,12Z)-Octadeca-9,12-dien-1-ol (Linoleylalkohol), ethoxyliert mit 113 EO,
- (9Z,12Z)-Octadeca-9,12-dien-1-ol (Linoleylalkohol), ethoxyliert mit 114 EO,
- (9Z,12Z)-Octadeca-9,12-dien-1-ol (Linoleylalkohol), ethoxyliert mit 115 EO,
- (9Z,12Z)-Octadeca-9,12-dien-1-ol (Linoleylalkohol), ethoxyliert mit 116 EO,
- (9Z,12Z)-Octadeca-9,12-dien-1-ol (Linoleylalkohol), ethoxyliert mit 117 EO,
- (9Z,12Z)-Octadeca-9,12-dien-1-ol (Linoleylalkohol), ethoxyliert mit 118 EO,
- (9Z,12Z)-Octadeca-9,12-dien-1-ol (Linoleylalkohol), ethoxyliert mit 119 EO,
- (9Z,12Z)-Octadeca-9,12-dien-1-ol (Linoleylalkohol), ethoxyliert mit 120 EO,
- Eicosan-1-ol (Eicosylalkohol, Arachylalkohol), ethoxyliert mit 50 EO,
- Eicosan-1-ol (Eicosylalkohol, Arachylalkohol), ethoxyliert mit 51 EO,
- Eicosan-1-ol (Eicosylalkohol, Arachylalkohol), ethoxyliert mit 52 EO,
- Eicosan-1-ol (Eicosylalkohol, Arachylalkohol), ethoxyliert mit 53 EO,
- Eicosan-1-ol (Eicosylalkohol, Arachylalkohol), ethoxyliert mit 54 EO,
- Eicosan-1-ol (Eicosylalkohol, Arachylalkohol), ethoxyliert mit 55 EO,
- Eicosan-1-ol (Eicosylalkohol, Arachylalkohol), ethoxyliert mit 56 EO,
- Eicosan-1-ol (Eicosylalkohol, Arachylalkohol), ethoxyliert mit 57 EO,
- Eicosan-1-ol (Eicosylalkohol, Arachylalkohol), ethoxyliert mit 58 EO,
- Eicosan-1-ol (Eicosylalkohol, Arachylalkohol), ethoxyliert mit 59 EO,
- Eicosan-1-ol (Eicosylalkohol, Arachylalkohol), ethoxyliert mit 60 EO,
- Eicosan-1-ol (Eicosylalkohol, Arachylalkohol), ethoxyliert mit 80 EO,
- Eicosan-1-ol (Eicosylalkohol, Arachylalkohol), ethoxyliert mit 81 EO,
- Eicosan-1-ol (Eicosylalkohol, Arachylalkohol), ethoxyliert mit 82 EO,
- Eicosan-1-ol (Eicosylalkohol, Arachylalkohol), ethoxyliert mit 83 EO,
- Eicosan-1-ol (Eicosylalkohol, Arachylalkohol), ethoxyliert mit 84 EO,
- Eicosan-1-ol (Eicosylalkohol, Arachylalkohol), ethoxyliert mit 85 EO,
- Eicosan-1-ol (Eicosylalkohol, Arachylalkohol), ethoxyliert mit 86 EO,
- Eicosan-1-ol (Eicosylalkohol, Arachylalkohol), ethoxyliert mit 87 EO,
- Eicosan-1-ol (Eicosylalkohol, Arachylalkohol), ethoxyliert mit 88 EO,
- Eicosan-1-ol (Eicosylalkohol, Arachylalkohol), ethoxyliert mit 89 EO,
- Eicosan-1-ol (Eicosylalkohol, Arachylalkohol), ethoxyliert mit 90 EO,
- Eicosan-1-ol (Eicosylalkohol, Arachylalkohol), ethoxyliert mit 91 EO,
- Eicosan-1-ol (Eicosylalkohol, Arachylalkohol), ethoxyliert mit 92 EO,
- Eicosan-1-ol (Eicosylalkohol, Arachylalkohol), ethoxyliert mit 93 EO,
- Eicosan-1-ol (Eicosylalkohol, Arachylalkohol), ethoxyliert mit 94 EO,
- Eicosan-1-ol (Eicosylalkohol, Arachylalkohol), ethoxyliert mit 95 EO,
- Eicosan-1-ol (Eicosylalkohol, Arachylalkohol), ethoxyliert mit 96 EO,
- Eicosan-1-ol (Eicosylalkohol, Arachylalkohol), ethoxyliert mit 97 EO,
- Eicosan-1-ol (Eicosylalkohol, Arachylalkohol), ethoxyliert mit 98 EO,
- Eicosan-1-ol (Eicosylalkohol, Arachylalkohol), ethoxyliert mit 99 EO,
- Eicosan-1-ol (Eicosylalkohol, Arachylalkohol), ethoxyliert mit 100 EO,
- Eicosan-1-ol (Eicosylalkohol, Arachylalkohol), ethoxyliert mit 101 EO,
- Eicosan-1-ol (Eicosylalkohol, Arachylalkohol), ethoxyliert mit 102 EO,
- Eicosan-1-ol (Eicosylalkohol, Arachylalkohol), ethoxyliert mit 103 EO,
- Eicosan-1-ol (Eicosylalkohol, Arachylalkohol), ethoxyliert mit 104 EO,
- Eicosan-1-ol (Eicosylalkohol, Arachylalkohol), ethoxyliert mit 105 EO,
- Eicosan-1-ol (Eicosylalkohol, Arachylalkohol), ethoxyliert mit 106 EO,
- Eicosan-1-ol (Eicosylalkohol, Arachylalkohol), ethoxyliert mit 107 EO,
- Eicosan-1-ol (Eicosylalkohol, Arachylalkohol), ethoxyliert mit 108 EO,
- Eicosan-1-ol (Eicosylalkohol, Arachylalkohol), ethoxyliert mit 109 EO,
- Eicosan-1-ol (Eicosylalkohol, Arachylalkohol), ethoxyliert mit 110 EO,
- Eicosan-1-ol (Eicosylalkohol, Arachylalkohol), ethoxyliert mit 111 EO,
- Eicosan-1-ol (Eicosylalkohol, Arachylalkohol), ethoxyliert mit 112 EO,
- Eicosan-1-ol (Eicosylalkohol, Arachylalkohol), ethoxyliert mit 113 EO,
- Eicosan-1-ol (Eicosylalkohol, Arachylalkohol), ethoxyliert mit 114 EO,
- Eicosan-1-ol (Eicosylalkohol, Arachylalkohol), ethoxyliert mit 115 EO,
- Eicosan-1-ol (Eicosylalkohol, Arachylalkohol), ethoxyliert mit 116 EO,
- Eicosan-1-ol (Eicosylalkohol, Arachylalkohol), ethoxyliert mit 117 EO,
- Eicosan-1-ol (Eicosylalkohol, Arachylalkohol), ethoxyliert mit 118 EO,
- Eicosan-1-ol (Eicosylalkohol, Arachylalkohol), ethoxyliert mit 119 EO,
- Eicosan-1-ol (Eicosylalkohol, Arachylalkohol), ethoxyliert mit 120 EO,
- Docosan-1-ol (Docosylalkohol, Behenylalkohol), ethoxyliert mit 50 EO
- Docosan-1-ol (Docosylalkohol, Behenylalkohol), ethoxyliert mit 51 EO
- Docosan-1-ol (Docosylalkohol, Behenylalkohol), ethoxyliert mit 52 EO
- Docosan-1-ol (Docosylalkohol, Behenylalkohol), ethoxyliert mit 53 EO
- Docosan-1-ol (Docosylalkohol, Behenylalkohol), ethoxyliert mit 54 EO
- Docosan-1-ol (Docosylalkohol, Behenylalkohol), ethoxyliert mit 55 EO
- Docosan-1-ol (Docosylalkohol, Behenylalkohol), ethoxyliert mit 56 EO
- Docosan-1-ol (Docosylalkohol, Behenylalkohol), ethoxyliert mit 57 EO
- Docosan-1-ol (Docosylalkohol, Behenylalkohol), ethoxyliert mit 58 EO
- Docosan-1-ol (Docosylalkohol, Behenylalkohol), ethoxyliert mit 59 EO
- Docosan-1-ol (Docosylalkohol, Behenylalkohol), ethoxyliert mit 60 EO
- Docosan-1-ol (Docosylalkohol, Behenylalkohol), ethoxyliert mit 80 EO
- Docosan-1-ol (Docosylalkohol, Behenylalkohol), ethoxyliert mit 81 EO
- Docosan-1-ol (Docosylalkohol, Behenylalkohol), ethoxyliert mit 82 EO
- Docosan-1-ol (Docosylalkohol, Behenylalkohol), ethoxyliert mit 83 EO
- Docosan-1-ol (Docosylalkohol, Behenylalkohol), ethoxyliert mit 84 EO
- Docosan-1-ol (Docosylalkohol, Behenylalkohol), ethoxyliert mit 85 EO
- Docosan-1-ol (Docosylalkohol, Behenylalkohol), ethoxyliert mit 86 EO
- Docosan-1-ol (Docosylalkohol, Behenylalkohol), ethoxyliert mit 87 EO
- Docosan-1-ol (Docosylalkohol, Behenylalkohol), ethoxyliert mit 88 EO
- Docosan-1-ol (Docosylalkohol, Behenylalkohol), ethoxyliert mit 89 EO
- Docosan-1-ol (Docosylalkohol, Behenylalkohol), ethoxyliert mit 90 EO
- Docosan-1-ol (Docosylalkohol, Behenylalkohol), ethoxyliert mit 91 EO
- Docosan-1-ol (Docosylalkohol, Behenylalkohol), ethoxyliert mit 92 EO
- Docosan-1-ol (Docosylalkohol, Behenylalkohol), ethoxyliert mit 93 EO
- Docosan-1-ol (Docosylalkohol, Behenylalkohol), ethoxyliert mit 94 EO
- Docosan-1-ol (Docosylalkohol, Behenylalkohol), ethoxyliert mit 95 EO
- Docosan-1-ol (Docosylalkohol, Behenylalkohol), ethoxyliert mit 96 EO
- Docosan-1-ol (Docosylalkohol, Behenylalkohol), ethoxyliert mit 97 EO
- Docosan-1-ol (Docosylalkohol, Behenylalkohol), ethoxyliert mit 98 EO
- Docosan-1-ol (Docosylalkohol, Behenylalkohol), ethoxyliert mit 99 EO
- Docosan-1-ol (Docosylalkohol, Behenylalkohol), ethoxyliert mit 100 EO
- Docosan-1-ol (Docosylalkohol, Behenylalkohol), ethoxyliert mit 101 EO
- Docosan-1-ol (Docosylalkohol, Behenylalkohol), ethoxyliert mit 102 EO
- Docosan-1-ol (Docosylalkohol, Behenylalkohol), ethoxyliert mit 103 EO
- Docosan-1-ol (Docosylalkohol, Behenylalkohol), ethoxyliert mit 104 EO
- Docosan-1-ol (Docosylalkohol, Behenylalkohol), ethoxyliert mit 105 EO
- Docosan-1-ol (Docosylalkohol, Behenylalkohol), ethoxyliert mit 106 EO
- Docosan-1-ol (Docosylalkohol, Behenylalkohol), ethoxyliert mit 107 EO
- Docosan-1-ol (Docosylalkohol, Behenylalkohol), ethoxyliert mit 108 EO
- Docosan-1-ol (Docosylalkohol, Behenylalkohol), ethoxyliert mit 109 EO
- Docosan-1-ol (Docosylalkohol, Behenylalkohol), ethoxyliert mit 110 EO
- Docosan-1-ol (Docosylalkohol, Behenylalkohol), ethoxyliert mit 111 EO
- Docosan-1-ol (Docosylalkohol, Behenylalkohol), ethoxyliert mit 112 EO
- Docosan-1-ol (Docosylalkohol, Behenylalkohol), ethoxyliert mit 113 EO
- Docosan-1-ol (Docosylalkohol, Behenylalkohol), ethoxyliert mit 114 EO
- Docosan-1-ol (Docosylalkohol, Behenylalkohol), ethoxyliert mit 115 EO
- Docosan-1-ol (Docosylalkohol, Behenylalkohol), ethoxyliert mit 116 EO
- Docosan-1-ol (Docosylalkohol, Behenylalkohol), ethoxyliert mit 117 EO
- Docosan-1-ol (Docosylalkohol, Behenylalkohol), ethoxyliert mit 118 EO
- Docosan-1-ol (Docosylalkohol, Behenylalkohol), ethoxyliert mit 119 EO
- Docosan-1-ol (Docosylalkohol, Behenylalkohol), ethoxyliert mit 120 EO enthält.

Eine ganz besonders vorteilhafte erfindungsgemäße Oxidationsusammensetzung zum Färben und/oder Aufhellen von keratinischen Fasern ist dadurch gekennzeichnet, dass sie als ethoxylierte(n) Fettalkohol(e) der Formel (IV) eine oder mehrere Verbindungen aus der Gruppe
- Hexadecan-1-ol (Hexadecylalkohol, Cetylalkohol, Palmitylalkohol), ethoxyliert mit 50 EO,
- Hexadecan-1-ol (Hexadecylalkohol, Cetylalkohol, Palmitylalkohol), ethoxyliert mit 51 EO,
- Hexadecan-1-ol (Hexadecylalkohol, Cetylalkohol, Palmitylalkohol), ethoxyliert mit 52 EO,
- Hexadecan-1-ol (Hexadecylalkohol, Cetylalkohol, Palmitylalkohol), ethoxyliert mit 53 EO,
- Hexadecan-1-ol (Hexadecylalkohol, Cetylalkohol, Palmitylalkohol), ethoxyliert mit 54 EO,
- Hexadecan-1-ol (Hexadecylalkohol, Cetylalkohol, Palmitylalkohol), ethoxyliert mit 55 EO,
- Hexadecan-1-ol (Hexadecylalkohol, Cetylalkohol, Palmitylalkohol), ethoxyliert mit 56 EO,
- Hexadecan-1-ol (Hexadecylalkohol, Cetylalkohol, Palmitylalkohol), ethoxyliert mit 57 EO,
- Hexadecan-1-ol (Hexadecylalkohol, Cetylalkohol, Palmitylalkohol), ethoxyliert mit 58 EO,
- Hexadecan-1-ol (Hexadecylalkohol, Cetylalkohol, Palmitylalkohol), ethoxyliert mit 59 EO,
- Hexadecan-1-ol (Hexadecylalkohol, Cetylalkohol, Palmitylalkohol), ethoxyliert mit 60 EO,
- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Octadecan-1-ol (Octadecylalkohol, Stearylalkohol), ethoxyliert mit 50 EO,
- Octadecan-1-ol (Octadecylalkohol, Stearylalkohol), ethoxyliert mit 51 EO,
- Octadecan-1-ol (Octadecylalkohol, Stearylalkohol), ethoxyliert mit 52 EO,
- Octadecan-1-ol (Octadecylalkohol, Stearylalkohol), ethoxyliert mit 53 EO,
- Octadecan-1-ol (Octadecylalkohol, Stearylalkohol), ethoxyliert mit 54 EO,
- Octadecan-1-ol (Octadecylalkohol, Stearylalkohol), ethoxyliert mit 55 EO,
- Octadecan-1-ol (Octadecylalkohol, Stearylalkohol), ethoxyliert mit 56 EO,
- Octadecan-1-ol (Octadecylalkohol, Stearylalkohol), ethoxyliert mit 57 EO,
- Octadecan-1-ol (Octadecylalkohol, Stearylalkohol), ethoxyliert mit 58 EO,
- Octadecan-1-ol (Octadecylalkohol, Stearylalkohol), ethoxyliert mit 59 EO,
- Octadecan-1-ol (Octadecylalkohol, Stearylalkohol), ethoxyliert mit 60 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,
enthält.

Als siebten wesentlichen Formulierungsbestandteil enthalten die erfindungsgemäßen Oxidationszusammensetzungen mindestens ein Polyol, ausgewählt aus C₂ - C₉-Alkanolen mit 2 - 6 Hydroxylgruppen und Polyethylenglycolen mit 3 - 20 Ethylenoxid-Einheiten sowie Mischungen hiervon, bevorzugt in einer Gesamtmenge von 1 - 10 Gew.-%, besonders bevorzugt 2 - 8 Gew.-%, außerordentlich bevorzugt 3 - 6 Gew.-%.

Erfindungsgemäß bevorzugte C₂ - C₉-Alkanole mit 2 - 6 Hydroxylgruppen und/oder Polyethylenglycole mit 3 - 20 Ethylenoxid-Einheiten sind ausgewählt aus 1,2-Propylenglycol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol und 2-Methyl-1,3-propandiol (INCI: Hexylene glycol), Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Als achten wesentlichen Formulierungsbestandteil enthalten die erfindungsgemäßen Oxidationszusammensetzungen mindestens ein Öl. Bevorzugt ist mindestens ein Öl in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Oxidationszusammensetzung, enthalten. Erfindungsgemäß bevorzugte Öle sind ausgewählt aus natürlichen und synthetischen Kohlenwasserstoffen, besonders bevorzugt aus Mineralölen, Paraffinölen, C₁₈-C₃₀-lsoparaffinen, insbesondere Isoeicosan, Polyisobutene und Polydecene, die beispielsweise unter der Bezeichnung Emery® 3004, 3006, 3010 oder unter der Bezeichnung Ethylflo® von Albemarle oder Nexbase® 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol® S von BASF).

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere bevorzugte Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Hexyldecylstearat (Eutanol® G 16 S), 2-Hexyldecyllaurat, Isodecylneopentanoat, Isononylisononanoat, 2-Ethylhexylpalmitat (Cegesoft® C 24) und 2-Ethylhexylstearat (Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß bevorzugte kosmetische Öle sind ausgewählt aus den C8-C22-Fettalkoholestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure. Solche Ester auf Basis von linearen C14/15-Alkanolen, z. B. C12-C15-Alkyllactat, und von in 2-Position verzweigten C12/13-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß bevorzugte kosmetische Öle sind ausgewählt aus den symmetrischen, unsymmetrischen oder cyclischen Estern der Kohlensäure mit C3-22-Alkanolen, C₃₋₂₂-Alkandiolen oder C₃₋₂₂-Alkantriolen, z. B. Dicaprylylcarbonat (Cetiol® CC) oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter C₁₂₋C₂₂-Fettsäuren (Dimerfettsäuren) mit einwertigen linearen, verzweigten oder cyclischen C₂₋C₁₈-Atkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Es kann erfindungsgemäß außerordentlich bevorzugt sein, Mischungen der vorgenannten Öle einzusetzen.

Bevorzugte erfindungsgemäße Oxidationszusammensetzungen sind dadurch gekennzeichnet, dass das kosmetische Öl ausgewählt ist aus natürlichen und synthetischen Kohlenwasserstoffen, besonders bevorzugt aus Paraffinölen, C₁₈₋C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈₋C₂₂-Fettalkoholestern einwertiger oder mehrwertiger C₂₋C₇-Hydroxycarbonsäuren; den symmetrischen, unsymmetrischen oder cyclischen Estern der Kohlensäure mit C₃-₂₂-Alkanolen, C₃₋₂₂-Alkandiolen oder C₃₋₂₂-Alkantriolen; den Estern von Dimeren ungesättigter C₁₂₋C₂₂-Fettsäuren (Dimerfettsäuren) mit einwertigen linearen, verzweigten oder cyclischen C₂₋C₁₈-Alkanolen oder mit mehrwertigen linearen oder verzweigten C₂₋C₆-Alkanolen; Siliconölen sowie Mischungen der vorgenannten Substanzen.

Erfindungsgemäße Oxidationszusammensetzungen sind dadurch gekennzeichnet, dass mindestens eine lamellare Phase enthalten ist. Die Inhaltsstoffe der erfindungsgemäßen Oxidationszusammensetzungen bilden bevorzugt mindestens eine lamellare Phase, bevorzugt eine L beta-Phase, was beim Vermischen mit einer Ammoniak-haltigen Alkalisierungszusammensetzung, bevorzugt beim Vermischen mit der erfindungsgemäß verwendeten Seifengelbasierten Alkalisierungszusammensetzung dazu führt, dass der in der Wasserphase gelöste Ammoniak im Wasser der lamellaren Phasen gebunden bleibt und so seine Verdampfung reduziert wird. Die erfindungsgemäße Oxidationszusammensetzung weist bevorzugt eine Viskosität im Bereich von 800 - 3500 mPas, besonders bevorzugt 1500 - 3000 mPas, auf, jeweils gemessen bei 20°C im Haake-Viskosimeter Typ MV2 mit einer Geschwindigkeit von 8 Umdrehungen/Minute.

Zur Stabilisierung des Oxidationsmittels während der Lagerung ist es insbesondere bevorzugt, dass die erfindungsgemäße Oxidationszusammensetzung einen sauren pH-Wert aufweist, insbesondere einen pH-Wert im Bereich von 2,5 bis 5,5, bevorzugt von 3,0 bis 5,0. Bevorzugte Acidifizierungsmittel sind Genusssäuren, wie beispielsweise Zitronensäure, Essigsäure, Äpfelsäure oder Weinsäure, sowie verdünnte Mineralsäuren, insbesondere Phosphorsäure.

Zur Stabilisierung des Oxidationsmittels in der erfindungsgemäßen Oxidationszusammensetzung ist es bevorzugt, so genannte Komplexbildner einzusetzen. Komplexbildner sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt. Die Zahl der gebundenen Liganden hängt von der Koordinationszahl des zentralen Ions ab. Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Auch Dipicolinsäure wird erfindungsgemäß vorzugsweise als Komplexbildner eingesetzt. Mittel, die eine Kombination aus einem EDTA-Salz und HEDP und Dipicolinsäure enthalten sind erfindungsgemäß besonders bevorzugt.

Ein weiterer Gegenstand der vorliegenden Anmeldung ist ein Kit zur oxidativen Farbveränderung keratinischer Fasern, enthaltend zwei voneinander getrennte Zusammensetzungen (A) und (B), wobei die Zusammensetzung (B) eine erfindungsgemäße oder erfindungsgemäß bevorzugte Oxidationszusammensetzung, wie vorstehend beschrieben ist, also eine Oxidationszusammensetzung, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
   - mindestens ein lineares gesättigtes 1-Alkanol mit 12 - 30 Kohlenstoffatomen enthalten ist, bevorzugt in einer Gesamtmenge von 2 - 8 Gew.-%, besonders bevorzugt 3 bis 6,5 Gew.-%,
   - mindestens einen Glycerylfettsäureester der allgemeinen Formel (I), 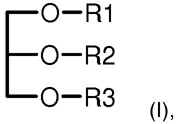 worin
   - R1, R2 und R3 unabhängig voneinander für ein Wasserstoffatom oder eine Gruppierung der Formel (II) stehen, 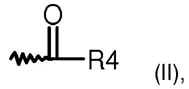 worin
   - R4 für eine unverzweigte oder verzweigte, gesättige oder ungesättigte C₁₁-C₂₇-Alkylgruppe steht,
   - mit der Maßgabe, dass mindestens einer und maximal zwei der Reste ausgewählt aus R1, R2 und R3 für eine Gruppierung der Formel (II) stehen,
      wobei der mindestens eine Glycerylfettsäureester der allgemeinen Formel (I) bevorzugt in einer Gesamtmenge von 0,01 - 1 Gew.-%, besonders bevorzugt 0,1 bis 0,8 Gew.-%, enthalten ist,
   - mindestens einen ethoxylierten Fettalkohol der Formel (III) 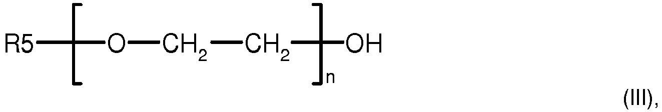 worin
      R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und n für eine ganze Zahl von 10 bis 30, bevorzugt für eine ganze Zahl von 12 bis 25, weiter bevorzugt für eine ganze Zahl von 15 bis 20 steht, wobei der mindestens eine ethoxylierte Fettalkohol der Formel (III) bevorzugt in einer Gesamtmenge von 0,25 - 2 Gew.-%, besonders bevorzugt 0,5 bis 1,5 Gew.-%, enthalten ist,
   - mindestens einen ethoxylierten Fettalkohol der Formel (IV) 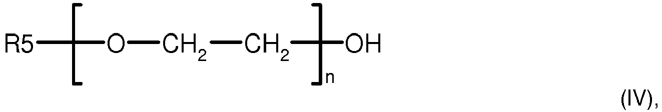 worin R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, und n für eine ganze Zahl von 50 bis 150, bevorzugt für eine ganze Zahl von 80 bis 120, weiter bevorzugt für die Zahl 100 steht, wobei der mindestens eine ethoxylierte Fettalkohol der Formel (IV) bevorzugt in einer Gesamtmenge von 0,03 - 0,3 Gew.-%, besonders bevorzugt 0,08 bis 0,25 Gew.-%, außerordentlich bevorzugt 0,1 - 0,2 Gew.-%, enthalten ist,
   - mindestens ein Polyol, ausgewählt aus C₂ - C₉-Alkanolen mit 2 - 6 Hydroxylgruppen und Polyethylenglycolen mit 3 - 20 Ethylenoxid-Einheiten sowie Mischungen hiervon, bevorzugt in einer Gesamtmenge von 1 - 10 Gew.-%, besonders bevorzugt 2 - 8 Gew.-%, außerordentlich bevorzugt 3 - 6 Gew.-%, und
   - mindestens ein Öl, bevorzugt in einer Gesamtmenge von 0,1 - 5 Gew.-%, besonders bevorzugt 0,2 - 3 Gew.-%, außerordentlich bevorzugt 0,5 - 2,5 Gew.-%,
   wobei alle Mengenangaben auf das Gewicht der Oxidationszusammensetzung bezogen sind, dadurch gekennzeichnet, dass mindestens eine lamellare Phase enthalten ist; und die Zusammensetzung (A) in Form eines Seifen-basierten Gels vorliegt und
   - 25 - 85 Gew.-% Wasser,
   - Ammoniak,
   - 5-20 Gew.-%, bevorzugt 8 - 16 Gew.-% mindestens eines Salzes einer C12-C22-Fettsäure, bevorzugt eines Salzes der Ölsäure,
   - 2-20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül,
   - 0 - 1 Gew.-% mindestens eines Öls,
   - bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-%,
   - optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül,
   - und
   - gegebenenfalls mindestens ein Oxidationsfarbstoffvorprodukt enthält und einen pH-Wert im Bereich von 8 bis 11,5, gemessen bei 20°C, aufweist,
   wobei alle Mengenangaben auf das Gewicht der Zusammensetzung (A) bezogen sind;
wobei die Zusammensetzungen (A) und (B) bevorzugt in einem gewichtsbezogenen Verhältnis (A)/(B) im Bereich von 0,33 - 3, besonders bevorzugt 0,5 - 2, außerordentlich bevorzugt 1:1, vorliegen.

Die Zusammensetzung (A) entspricht der vorstehend beschriebenen Alkalisierungszubereitung. Die Zusammensetzung (A) enthält Ammoniak und optional mindestens ein weiteres Alkalisierungsmittel, das ausgewählt ist aus der Gruppe, die gebildet wird von Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten. Geeignete anorganische Alkalisierungsmittel sind Natriumhydroxid, Kaliumhydroxid, Natriumsilicat und Natriummetasilicat. Erfindungsgemäß einsetzbare, organische Alkalisierungsmittel sind ausgewählt aus Monoethanolamin, 2-Amino-2-methylpropanol und Triethanolamin. Die als erfindungsgemäßes Alkalisierungsmittel einsetzbaren basischen Aminosäuren sind bevorzugt ausgewählt aus der Gruppe, die gebildet wird aus Arginin, Lysin, Ornithin und Histidin, besonders bevorzugt Arginin. Üblicherweise wird Ammoniak (NH₃) in Form seiner wässrigen Lösung eingesetzt. Wässrige Ammoniak-Lösungen enthalten Ammoniak (NH₃) oft in Konzentrationen von 10 bis 32 Gew.-%. Bevorzugt ist hierbei der Einsatz einer wässrigen Ammoniak-Lösung, die 25 Gew.-% Ammoniak (NH₃) enthält. Bevorzugt ist die Gesamtmenge an Alkalisierungsmitteln so gewählt, dass die Mischung, also das anwendungsbereite Farbveränderungsmittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist. Bevorzugt sind Ammoniak und ggf. Monoethanolamin in der erfindungsgemäß verwendeten Zusammensetzung (A) in Mengen von 0,01 - 10 Gew.-%, bevorzugt von 0,1 bis 7,5 Gew.-%, weiter bevorzugt von 0,2 bis 5,5 Gew.-% und besonders bevorzugt von 0,4 bis 4,5 Gew.-% - jeweils bezogen auf das Gewicht der Zusammensetzung (A) - enthalten, so dass der pH-Wert der Zusammensetzung (A) im Bereich von 8 bis 11,5 liegt, gemessen bei 20°C.

Als optionalen Inhaltsstoff enthält die erfindungsgemäß verwendete Alkalisierungszusammensetzung (A) mindestens ein Oxidationsfarbstoffvorprodukt, das vorzugsweise aus einer oder mehreren Entwicklerkomponenten und gegebenenfalls einer oder mehreren Kupplerkomponenten ausgewählt ist.

Bevorzugt ist mindestens ein Oxidationsfarbstoffvorprodukt in einer Gesamtmenge von 0,0001 bis 10,0 Gew.-%, bevorzugt 0,001 bis 8 Gew.-%, jeweils bezogen auf das Gewicht der Zusammensetzung (A), enthalten.

Es kann erfindungsgemäß bevorzugt sein, als Entwicklerkomponente mindestens eine Verbindung aus der Gruppe auszuwählen, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Bevorzugt ist mindestens eine Entwicklerkomponente in einer Gesamtmenge von 0,0001 bis 10,0 Gew.-%, bevorzugt 0,001 bis 8 Gew.-%, jeweils bezogen auf das Gewicht der Zusammensetzung (A), enthalten.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Erfindungsgemäß bevorzugte Kupplerkomponenten sind ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Bevorzugt ist mindestens eine Kupplerkomponente in einer Gesamtmenge von 0,0001 bis 10,0 Gew.-%, bevorzugt 0,001 bis 8 Gew.-%, jeweils bezogen auf das Gewicht der Zusammensetzung (A), enthalten.

Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Erfindungsgemäß bevorzugte Kits zur oxidativen Farbveränderung keratinischer Fasern sind dadurch gekennzeichnet, dass die vorgenannte erfindungsgemäße oder erfindungsgemäß bevorzugte Oxidationszusammensetzung (B) und die vorgenannte Alkalisierungszusammensetzung (A) in einem Gewichtsverhältnis A/B von 0,33 bis 3, besonders bevorzugt von 0,5 bis 2 und außerordentlich bevorzugt im Gewichtsverhältnis von 1 :1, enthalten sind.

Erfindungsgemäß besonders bevorzugte Kits zur oxidativen Farbveränderung keratinischer Fasern sind dadurch gekennzeichnet, dass die vorgenannte erfindungsgemäße oder erfindungsgemäß bevorzugte Oxidationszusammensetzung (B) und die vorgenannte Alkalisierungszusammensetzung (A) in einem Gewichtsverhältnis A/B von 0,33 bis 3, besonders bevorzugt von 0,5 bis 2 und außerordentlich bevorzugt im Gewichtsverhältnis von 1 :1, enthalten sind, wobei das Kit keine weiteren Komponenten enthält, die zur anwendungsbereiten Farbveränderungsmischung zugegeben werden, während Komponenten zur Vor- oder Nachbehandlung der keratinischen Fasern, beispielsweise Conditioner oder Shampoos, im Kit enthalten sein können.

In einer erfindungsgemäß bevorzugten Ausführungsform ist das erfindungsgemäße Kit dadurch gekennzeichnet, dass die Zusammensetzung (A), jeweils bezogen auf ihr Gewicht, 25 - 85 Gew.-% Wasser, Ammoniak, 5-20 Gew.-%, bevorzugt 8 - 16 Gew.-% mindestens eines Salzes einer C12-C22-Fettsäure, bevorzugt eines Salzes der Ölsäure, optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül, 2 - 20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül, 0 - 1 Gew.-% mindestens eines Öls, bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-% und gegebenenfalls mindestens ein Oxidationsfarbstoffvorprodukt enthält und einen pH-Wert im Bereich von 8 bis 11,5, gemessen bei 20°C, aufweist, enthält.

Bezüglich weiterer bevorzugter Ausführungsformen des erfindungsgemäßen Kits gilt mutatis mutandis das zu den erfindungsgemäßen und erfindungsgemäß bevorzugten Oxidationszusammensetzungen sowie das zu den erfindungsgemäß verwendeten Alkalisierungszusammensetzungen (A) Gesagte.

Ein weiterer Gegenstand der vorliegenden Anmeldung ist ein Verfahren zur oxidativen Farbveränderung keratinischer Fasern, das durch folgende Verfahrensschritte gekennzeichnet ist:
Bereitstellung einer Oxidationszusammensetzung (B) gemäß einem der Ansprüche 1 - 7, enthaltend Bereitstellung einer Oxidationszusammensetzung (B) gemäß einem der Ansprüche 1 - 7, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens ein lineares gesättigtes 1-Alkanol mit 12 - 30 Kohlenstoffatomen enthalten ist, bevorzugt in einer Gesamtmenge von 2 - 8 Gew.-%, besonders bevorzugt 3 bis 6,5 Gew.-%,
- mindestens einen Glycerylfettsäureester der allgemeinen Formel (I), 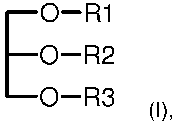 worin
- R1, R2 und R3 unabhängig voneinander für ein Wasserstoffatom oder eine Gruppierung der Formel (II) stehen, 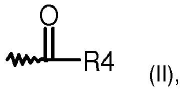 worin
- R4 für eine unverzweigte oder verzweigte, gesättige oder ungesättigte C₁₁-C₂₇-Alkylgruppe steht,
- mit der Maßgabe, dass mindestens einer und maximal zwei der Reste ausgewählt aus R1, R2 und R3 für eine Gruppierung der Formel (II) stehen,
   wobei der mindestens eine Glycerylfettsäureester der allgemeinen Formel (I) bevorzugt in einer Gesamtmenge von 0,01 - 1 Gew.-%, besonders bevorzugt 0,1 bis 0,8 Gew.-%, enthalten ist,
- mindestens einen ethoxylierten Fettalkohol der Formel (III) 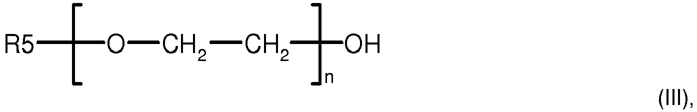 worin
   R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und n für eine ganze Zahl von 10 bis 30, bevorzugt für eine ganze Zahl von 12 bis 25, weiter bevorzugt für eine ganze Zahl von 15 bis 20 steht, wobei der mindestens eine ethoxylierte Fettalkohol der Formel (III) bevorzugt in einer Gesamtmenge von 0,25 - 2 Gew.-%, besonders bevorzugt 0,5 bis 1,5 Gew.-%, enthalten ist,
- mindestens einen ethoxylierten Fettalkohol der Formel (IV) 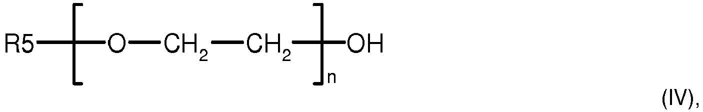 worin
   R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, und n für eine ganze Zahl von 50 bis 150, bevorzugt für eine ganze Zahl von 80 bis 120, weiter bevorzugt für die Zahl 100 steht, wobei der mindestens eine ethoxylierte Fettalkohol der Formel (IV) bevorzugt in einer Gesamtmenge von 0,03 - 0,3 Gew.-%, besonders bevorzugt 0,08 bis 0,25 Gew.-%, außerordentlich bevorzugt 0,1 - 0,2 Gew.-%, enthalten ist,
- mindestens ein Polyol, ausgewählt aus C₂ - C₉-Alkanolen mit 2 - 6 Hydroxylgruppen und Polyethylenglycolen mit 3 - 20 Ethylenoxid-Einheiten sowie Mischungen hiervon, bevorzugt in einer Gesamtmenge von 1 - 10 Gew.-%, besonders bevorzugt 2 - 8 Gew.-%, außerordentlich bevorzugt 3 - 6 Gew.-%, und
- mindestens ein Öl, bevorzugt in einer Gesamtmenge von 0,1 - 5 Gew.-%, besonders bevorzugt 0,2 - 3 Gew.-%, außerordentlich bevorzugt 0,5 - 2,5 Gew.-%,
   wobei alle Mengenangaben auf das Gewicht der Oxidationszusammensetzung bezogen sind, dadurch gekennzeichnet, dass mindestens eine lamellare Phase enthalten ist, und Bereitstellung einer Zusammensetzung (A), die in Form eines Seifen-basierten Gels vorliegt und
- 25 - 85 Gew.-% Wasser,
- Ammoniak,
- 5 - 20 Gew.-%, bevorzugt 8 - 16 Gew.-% mindestens eines Salzes einer C12-C22-Fettsäure, bevorzugt eines Salzes der Ölsäure,
- 2 - 20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül,
- insgesamt 0 - 1 Gew.-% mindestens eines Öls,
- bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-%
- optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül, und
- gegebenenfalls mindestens ein Oxidationsfarbstoffvorprodukt
   enthält und einen pH-Wert im Bereich von 8 bis 11,5, gemessen bei 20°C, aufweist,
wobei alle Mengenangaben auf das Gewicht der Zusammensetzung (A) bezogen sind; Herstellung einer Mischung aus der vorgenannten Oxidationszusammensetzung (B) und der vorgenannten Zusammensetzung (A), bevorzugt im gewichtsbezogenen Mischungsverhältnis (A)/(B) im Bereich von 0,33 - 3, besonders bevorzugt 0,5 - 2, außerordentlich bevorzugt 1:1, unmittelbar danach Verteilung des anwendungsbereiten Mittels auf den Fasern,

Verbleib des Mittels auf den Fasern für einen Zeitraum von 1 bis 60 Minuten, unmittelbar danach Ausspülen des verbliebenen Mittels aus den Fasern und ggf. Trocknen der Fasern.

Erfindungsgemäß bevorzugte Verfahren zur oxidativen Farbveränderung keratinischer Fasern sind dadurch gekennzeichnet, dass die vorgenannte Oxidationszusammensetzung (B) und die vorgenannte Alkalisierungszusammensetzung (A) im gewichtsbezogenen Mischungsverhältnis (A)/(B) im Bereich von 0,33 - 3, besonders bevorzugt 0,5 - 2, außerordentlich bevorzugt 1:1, miteinander vermischt werden.

Bezüglich weiterer bevorzugter Ausführungsformen des erfindungsgemäßen Verfahrens gilt mutatis mutandis das zu den erfindungsgemäßen und erfindungsgemäß bevorzugten Oxidationszusammensetzungen sowie das zu den erfindungsgemäß verwendeten Alkalisierungszusammensetzungen (A) Gesagte.

Unter keratinischen bzw. keratinhaltigen Fasern sind erfindungsgemäß Wolle, Pelze, Federn und insbesondere menschliche Haare zu verstehen. Die erfindungsgemäßen Färbe- und/oder Aufhellverfahren können prinzipiell aber auch zur Anwendung auf anderen Naturfasern, wie z. B. Baumwolle, Jute, Sisal, Leinen, Seide oder modifizierten Naturfasern, wie beispielsweise Regeneratcellulose, Nitro-, Alkyl- oder Hydroxyalkyl- oder Acetylcellulose, verwendet werden.

Das anwendungsbereite Färbemittel des erfindungsgemäßen Verfahrens wird bevorzugt durch Zusammenführen der erfindungsgemäßen oder erfindungsgemäß bevorzugten Oxidationszusammensetzung mit einer erfindungsgemäß verwendeten Alkalisierungszusammensetzung (A) in einem wiederverschließbaren Behältnis und anschließendes Vermischen hergestellt.

Im anschließenden Verfahrensschritt wird das anwendungsbereite Färbemittel auf den keratinischen Fasern verteilt. Bei dem Verfahren zur Farbveränderung menschlicher Haare wird das anwendungsbereite Mittel dabei direkt auf dem Kopfhaar des Anwenders verteilt. Bevorzugt erfolgt die Verteilung manuell. Dazu entnimmt der Anwender das anwendungsbereite Mittel dem Mischungsbehältnis, bevorzugt dem wiederverschließbaren Behältnis, durch Schöpfen oder Gießen auf die Hand und anschließende Verteilung und bevorzugt Einarbeitung des Mittels auf dem Kopfhaar. Bevorzugt wird dabei der direkte Kontakt zwischen anwendungsbereitem Farbveränderungsmittel und den Händen durch die Verwendung von geeigneten Handschuhen, wie Einweghandschuhen, beispielsweise aus Latex, vermieden.

Anschließend verbleibt das anwendungsbereite Färbemittel für einen Zeitraum von 1 bis 60 min auf den zu behandelnden Fasern. Bevorzugt liegt der Zeitraum im Bereich von 10 bis 45 min, besonders bevorzugt 20 bis 30 Minuten.

Die Anwendungstemperaturen können in einem Bereich zwischen 15 und 40 °C liegen. Gegebenenfalls kann während der Verbleibzeit des Mittels auf den Fasern auch durch externe Wärmequellen eine höhere oder genau definierte Temperatur eingestellt werden. Besonders bevorzugt ist es, die Farbveränderung durch physikalische Maßnahmen zu unterstützen. Erfindungsgemäße Verfahren, bei denen die Anwendung durch Einwirkung von Wärme, IR- und/oder UV-Strahlung während der Einwirkzeit unterstützt wird, können dabei bevorzugt sein.

Nach Ablauf der Einwirkungszeit wird das anwendungsbereite Färbemittel bzw. das verbleibende Färbemittel im letzten Verfahrensschritt durch Ausspülen von den zu behandelnden Fasern entfernt. Hierzu werden die Fasern mit Wasser und/oder einer wässrigen Tensid-Zubereitung ausgespült. Üblicherweise wird hierzu 20 °C bis 40 °C warmes Wasser bzw. eine entsprechend temperierte wässrige Tensid-Zubereitung eingesetzt. Optional können sich hieran weitere Behandlungsschritte anschließen, wie das Auftragen eines Leave-on oder Rinse-off-Conditioners, ein weiterer Färbeschritt, z.B. das Einfärben oder Aufhellen von Strähnchen, eine Haarverformung und/oder das Trocknen der Haare.

## Patentansprüche

1. Oxidationszusammensetzung zur oxidativen Haarbehandlung, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens ein lineares gesättigtes 1-Alkanol mit 12 - 30 Kohlenstoffatomen, bevorzugt in einer Gesamtmenge von 2 - 8 Gew.-%, besonders bevorzugt 3 bis 6,5 Gew.-%,
- mindestens einen Glycerylfettsäureester der allgemeinen Formel (I), 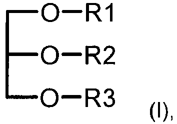 worin
- R1, R2 und R3 unabhängig voneinander für ein Wasserstoffatom oder eine Gruppierung der Formel (II) stehen, 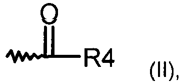 worin
- R4 für eine unverzweigte oder verzweigte, gesättige oder ungesättigte C₁₁-C₂₇-Alkylgruppe steht,
- mit der Maßgabe, dass mindestens einer und maximal zwei der Reste ausgewählt aus R1, R2 und R3 für eine Gruppierung der Formel (II) stehen,
wobei der mindestens eine Glycerylfettsäureester der allgemeinen Formel (I) bevorzugt in einer Gesamtmenge von 0,01 - 1 Gew.-%, besonders bevorzugt 0,1 bis 0,8 Gew.-%, enthalten ist,
- mindestens einen ethoxylierten Fettalkohol der Formel (III) 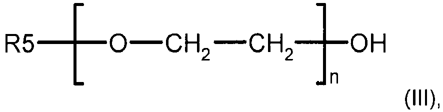 worin
R5 für eine gesättigte oder ungesättigte, unverzweigte oder verzweigte C₈-C₂₄-Alkylgruppe, bevorzugt für eine gesättigte, unverzweigte C₁₆- oder C₁₈ - Alkylgruppe, steht und n für eine ganze Zahl von 10 bis 30, bevorzugt für eine ganze Zahl von 12 bis 25, weiter bevorzugt für eine ganze Zahl von 15 bis 20 steht,
wobei der mindestens eine ethoxylierte Fettalkohol der Formel (III) bevorzugt in einer Gesamtmenge von 0,25 - 2 Gew.-%, besonders bevorzugt 0,5 bis 1,5 Gew.-%, enthalten ist,
- mindestens einen ethoxylierten Fettalkohol der Formel (IV) 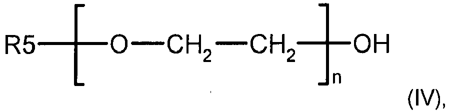 worin
R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, und n für eine ganze Zahl von 50 bis 150, bevorzugt für eine ganze Zahl von 80 bis 120, weiter bevorzugt für die Zahl 100 steht,
wobei der mindestens eine ethoxylierte Fettalkohol der Formel (IV) bevorzugt in einer Gesamtmenge von 0,03 - 0,3 Gew.-%, besonders bevorzugt 0,08 bis 0,25 Gew.-%, außerordentlich bevorzugt 0,1 - 0,2 Gew.-%, enthalten ist,
- mindestens ein Polyol, ausgewählt aus C₂ - C₉-Alkanolen mit 2 - 6 Hydroxylgruppen und Polyethylenglycolen mit 3 - 20 Ethylenoxid-Einheiten sowie Mischungen hiervon, bevorzugt in einer Gesamtmenge von 1 - 10 Gew.-%, besonders bevorzugt 2 - 8 Gew.-%, außerordentlich bevorzugt 3 - 6 Gew.-%, und
- mindestens ein Öl, bevorzugt in einer Gesamtmenge von 0,1 - 5 Gew.-%, besonders bevorzugt 0,2 - 3 Gew.-%, außerordentlich bevorzugt 0,5 - 2,5 Gew.-%,
wobei alle Mengenangaben auf das Gewicht der Oxidationszusammensetzung bezogen sind,
**dadurch gekennzeichnet, dass** mindestens eine lamellare Phase enthalten ist.

2. Zusammensetzung gemäß Anspruch 1, **dadurch gekennzeichnet, dass** das mindestens eine lineare gesättigte 1-Alkanol mit 12-30 Kohlenstoffatomen ausgewählt ist aus Laurylalkohol, Myristylalkohol, Cetylalkohol, Stearylalkohol, Arachidylalkohol und Behenylalkohol sowie aus Mischungen dieser Alkanole, bevorzugt aus Cetylalkohol, Stearylalkohol, Arachidylalkohol, Behenylalkohol und Cetylalkohol/ Stearylalkohol-Mischungen.

3. Zusammensetzung gemäß Anspruch 1 oder 2, **dadurch gekennzeichnet, dass** als Glycerylfettsäureester der Formel (I) mindestens eine Verbindung, ausgewählt aus Glycerylmonostearat, Glyceryldistearat, Glycerylmonopalmitat und Glyceryldipalmitat, in einer Gesamtmenge von 0,01 bis 1 Gew.-%, bevorzugt von 0,1 bis 0,8 Gew.-%, bezogen auf das Gewicht der erfindungsgemäßen Oxidationszusammensetzung, enthalten ist.

4. Zusammensetzung gemäß einem der Ansprüche 1 bis 3, **dadurch gekennzeichnet, dass** als ethoxylierter Fettalkohol der Formel (III) mindestens eine Verbindung, ausgewählt aus
- Decan-1-ol (Decylalkohol), ethoxyliert mit 10 EO,
- Decan-1-ol (Decylalkohol), ethoxyliert mit 20 EO,
- Decan-1-ol (Decylalkohol), ethoxyliert mit 30 EO,
- Dodecan-1-ol (Dodecylalkohol, Laurylalkohol), ethoxyliert mit 10 EO,
- Dodecan-1-ol (Dodecylalkohol, Laurylalkohol), ethoxyliert mit 20 EO,
- Dodecan-1-ol (Dodecylalkohol, Laurylalkohol), ethoxyliert mit 30 EO,
- Hexadecan-1-ol (Hexadecylalkohol, Cetylalkohol, Palmitylalkohol), ethoxyliert mit 10 EO,
- Hexadecan-1-ol (Hexadecylalkohol, Cetylalkohol, Palmitylalkohol), ethoxyliert mit 12 EO,
- Hexadecan-1-ol (Hexadecylalkohol, Cetylalkohol, Palmitylalkohol), ethoxyliert mit 15 EO,
- Hexadecan-1-ol (Hexadecylalkohol, Cetylalkohol, Palmitylalkohol), ethoxyliert mit 20 EO,
- Hexadecan-1-ol (Hexadecylalkohol, Cetylalkohol, Palmitylalkohol), ethoxyliert mit 25 EO,
- Hexadecan-1-ol (Hexadecylalkohol, Cetylalkohol, Palmitylalkohol), ethoxyliert mit 30 EO,
- Octadecan-1-ol (Octadecylalkohol, Stearylalkohol), ethoxyliert mit 10 EO,
- Octadecan-1-ol (Octadecylalkohol, Stearylalkohol), ethoxyliert mit 12 EO,
- Octadecan-1-ol (Octadecylalkohol, Stearylalkohol), ethoxyliert mit 15 EO,
- Octadecan-1-ol (Octadecylalkohol, Stearylalkohol), ethoxyliert mit 20 EO,
- Octadecan-1-ol (Octadecylalkohol, Stearylalkohol), ethoxyliert mit 25 EO,
- Octadecan-1-ol (Octadecylalkohol, Stearylalkohol), ethoxyliert mit 30 EO,
- Eicosan-1-ol (Eicosylalkohol, Arachylalkohol), ethoxyliert mit 10 EO,
- Eicosan-1-ol (Eicosylalkohol, Arachylalkohol), ethoxyliert mit 12 EO,
- Eicosan-1-ol (Eicosylalkohol, Arachylalkohol), ethoxyliert mit 15 EO,
- Eicosan-1-ol (Eicosylalkohol, Arachylalkohol), ethoxyliert mit 20 EO,
- Eicosan-1-ol (Eicosylalkohol, Arachylalkohol), ethoxyliert mit 25 EO,
- Eicosan-1-ol (Eicosylalkohol, Arachylalkohol), ethoxyliert mit 30 EO,
- Docosan-1-ol (Docosylalkohol, Behenylalkohol), ethoxyliert mit 10 EO
- Docosan-1-ol (Docosylalkohol, Behenylalkohol), ethoxyliert mit 12 EO
- Docosan-1-ol (Docosylalkohol, Behenylalkohol), ethoxyliert mit 15 EO
- Docosan-1-ol (Docosylalkohol, Behenylalkohol), ethoxyliert mit 20 EO
- Docosan-1-ol (Docosylalkohol, Behenylalkohol), ethoxyliert mit 25 EO
- Docosan-1-ol (Docosylalkohol, Behenylalkohol), ethoxyliert mit 30 EO,
sowie Mischungen hiervon
enthalten ist.

5. Zusammensetzung gemäß einem der Ansprüche 1 bis 4, **dadurch gekennzeichnet, dass** als ethoxylierter Fettalkohol der Formel (IV) mindestens eine Verbindung, ausgewählt aus
- Hexadecan-1-ol (Hexadecylalkohol, Cetylalkohol, Palmitylalkohol), ethoxyliert mit 50 EO,
- Hexadecan-1-ol (Hexadecylalkohol, Cetylalkohol, Palmitylalkohol), ethoxyliert mit 51 EO,
- Hexadecan-1-ol (Hexadecylalkohol, Cetylalkohol, Palmitylalkohol), ethoxyliert mit 52 EO,
- Hexadecan-1-ol (Hexadecylalkohol, Cetylalkohol, Palmitylalkohol), ethoxyliert mit 53 EO,
- Hexadecan-1-ol (Hexadecylalkohol, Cetylalkohol, Palmitylalkohol), ethoxyliert mit 54 EO,
- Hexadecan-1-ol (Hexadecylalkohol, Cetylalkohol, Palmitylalkohol), ethoxyliert mit 55 EO,
- Hexadecan-1-ol (Hexadecylalkohol, Cetylalkohol, Palmitylalkohol), ethoxyliert mit 56 EO,
- Hexadecan-1-ol (Hexadecylalkohol, Cetylalkohol, Palmitylalkohol), ethoxyliert mit 57 EO,
- Hexadecan-1-ol (Hexadecylalkohol, Cetylalkohol, Palmitylalkohol), ethoxyliert mit 58 EO,
- Hexadecan-1-ol (Hexadecylalkohol, Cetylalkohol, Palmitylalkohol), ethoxyliert mit 59 EO,
- Hexadecan-1-ol (Hexadecylalkohol, Cetylalkohol, Palmitylalkohol), ethoxyliert mit 60 EO,
- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Octadecan-1-ol (Octadecylalkohol, Stearylalkohol), ethoxyliert mit 50 EO,
- Octadecan-1-ol (Octadecylalkohol, Stearylalkohol), ethoxyliert mit 51 EO,
- Octadecan-1-ol (Octadecylalkohol, Stearylalkohol), ethoxyliert mit 52 EO,
- Octadecan-1-ol (Octadecylalkohol, Stearylalkohol), ethoxyliert mit 53 EO,
- Octadecan-1-ol (Octadecylalkohol, Stearylalkohol), ethoxyliert mit 54 EO,
- Octadecan-1-ol (Octadecylalkohol, Stearylalkohol), ethoxyliert mit 55 EO,
- Octadecan-1-ol (Octadecylalkohol, Stearylalkohol), ethoxyliert mit 56 EO,
- Octadecan-1-ol (Octadecylalkohol, Stearylalkohol), ethoxyliert mit 57 EO,
- Octadecan-1-ol (Octadecylalkohol, Stearylalkohol), ethoxyliert mit 58 EO,
- Octadecan-1-ol (Octadecylalkohol, Stearylalkohol), ethoxyliert mit 59 EO,
- Octadecan-1-ol (Octadecylalkohol, Stearylalkohol), ethoxyliert mit 60 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecän-1ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,
enthalten ist.

6. Zusammensetzung gemäß einem der Ansprüche 1 bis 5, **dadurch gekennzeichnet, dass** das mindestens eine Polyol ausgewählt ist aus ausgewählt aus 1,2-Propylenglycol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol und 2-Methyl-1,3-propandiol (INCI: Hexylene glycol), Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, PEG-3, PEG-4, PEG-6, PEG-7, PEG-8, sowie Mischungen der vorgenannten Substanzen.

7. Zusammensetzung gemäß einem der Ansprüche 1 bis 6, **dadurch gekennzeichnet, dass** das mindestens eine Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂₋C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

8. Kit zur oxidativen Farbveränderung keratinischer Fasern, enthaltend zwei voneinander getrennte Zusammensetzungen (A) und (B), wobei
die Zusammensetzung (B) eine Oxidationszusammensetzung nach einem der Ansprüche 1 - 7 ist und
die Zusammensetzung (A) in Form eines Seifen-basierten Gels vorliegt und
- 25 - 85 Gew.-% Wasser,
- Ammoniak,
- 5 - 20 Gew.-%, bevorzugt 8 - 16 Gew.-% mindestens eines Salzes einer C12-C22-Fettsäure, bevorzugt eines Salzes der Ölsäure,
- 2 - 20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül,
- insgesamt 0 - 1 Gew.-% mindestens eines Öls,
- bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-%,
- optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül, und
- gegebenenfalls mindestens ein Oxidationsfarbstoffvorprodukt
enthält und einen pH-Wert im Bereich von 8 bis 11,5, gemessen bei 20°C, aufweist,
wobei alle Mengenangaben auf das Gewicht der Zusammensetzung (A) bezogen sind;
wobei die Zusammensetzungen (A) und (B) bevorzugt in einem gewichtsbezogenen Verhältnis (A)/(B) im Bereich von 0,33 - 3, besonders bevorzugt 0,5 - 2, außerordentlich bevorzugt 1:1, vorliegen.

9. Verfahren zur oxidativen Farbveränderung keratinischer Fasern, **gekennzeichnet durch** folgende Verfahrensschritte:
Bereitstellung einer Oxidationszusammensetzung (B) gemäß einem der Ansprüche 1 - 7, enthaltend
- 50 - 96 Gew.-%, bevorzugt 70 - 93 Gew.-%, besonders bevorzugt 80 - 90 Gew.-%, Wasser,
- 0,5 - 20 Gew.-% Wasserstoffperoxid,
- mindestens ein lineares gesättigtes 1-Alkanol mit 12 - 30 Kohlenstoffatomen, bevorzugt in einer Gesamtmenge von 2 - 8 Gew.-%, besonders bevorzugt 3 bis 6,5 Gew.-%,
- mindestens einen Glycerylfettsäureester der allgemeinen Formel (I), 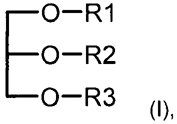 worin
- R1, R2 und R3 unabhängig voneinander für ein Wasserstoffatom oder eine Gruppierung der Formel (II) stehen, 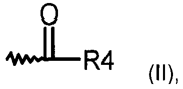 worin
- R4 für eine unverzweigte oder verzweigte, gesättige oder ungesättigte C₁₁-C₂₇-Alkylgruppe steht,
- mit der Maßgabe, dass mindestens einer und maximal zwei der Reste ausgewählt aus R1, R2 und R3 für eine Gruppierung der Formel (II) stehen,
wobei der mindestens eine Glycerylfettsäureester der allgemeinen Formel (I) bevorzugt in einer Gesamtmenge von 0,01 - 1 Gew.-%, besonders bevorzugt 0,1 bis 0,8 Gew.-%, enthalten ist,
- mindestens einen ethoxylierten Fettalkohol der Formel (III) 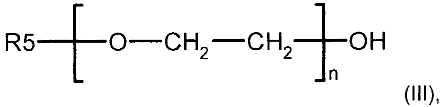 worin
R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, steht und n für eine ganze Zahl von 10 bis 30, bevorzugt für eine ganze Zahl von 12 bis 25, weiter bevorzugt für eine ganze Zahl von 15 bis 20 steht,
wobei der mindestens eine ethoxylierte Fettalkohol der Formel (III) bevorzugt in einer Gesamtmenge von 0,25 - 2 Gew.-%, besonders bevorzugt 0,5 bis 1,5 Gew.-%, enthalten ist,
- mindestens einen ethoxylierten Fettalkohol der Formel (IV) 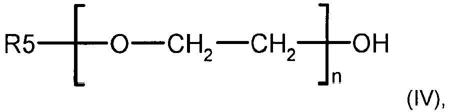 worin
R5 für eine gesättigte oder ungesättigte, unverzweigte oder verzweigte C₈-C₂₄-Alkylgruppe, bevorzugt für eine gesättigte, unverzweigte C₁₆- oder C₁₈- Alkylgruppe, und n für eine ganze Zahl von 50 bis 150, bevorzugt für eine ganze Zahl von 80 bis 120, weiter bevorzugt für die Zahl 100 steht,
wobei der mindestens eine ethoxylierte Fettalkohol der Formel (IV) bevorzugt in einer Gesamtmenge von 0,03 - 0,3 Gew.-%, besonders bevorzugt 0,08 bis 0,25 Gew.-%, außerordentlich bevorzugt 0,1 - 0,2 Gew.-%, enthalten ist,
- mindestens ein Polyol, ausgewählt aus C₂ - C₉-Alkanolen mit 2 - 6 Hydroxylgruppen und Polyethylenglycolen mit 3 - 20 Ethylenoxid-Einheiten sowie Mischungen hiervon, bevorzugt in einer Gesamtmenge von 1 - 10 Gew.-%, besonders bevorzugt 2 - 8 Gew.-%, außerordentlich bevorzugt 3 - 6 Gew.-%, und
- mindestens ein Öl, bevorzugt in einer Gesamtmenge von 0,1 - 5 Gew.-%, besonders bevorzugt 0,2 - 3 Gew.-%, außerordentlich bevorzugt 0,5 - 2,5 Gew.-%,
wobei alle Mengenangaben auf das Gewicht der Oxidationszusammensetzung bezogen sind, **dadurch gekennzeichnet, dass** mindestens eine lamellare Phase enthalten ist, und Bereitstellung einer Zusammensetzung (A), die in Form eines Seifen-basierten Gels vorliegt und
- 25 - 85 Gew.-% Wasser,
- Ammoniak,
- 5 - 20 Gew.-%, bevorzugt 8 - 16 Gew.-% mindestens eines Salzes einer C12-C22-Fettsäure, bevorzugt eines Salzes der Ölsäure,
- 2 - 20 Gew.-%, bevorzugt 3 - 16 Gew.-%, mindestens eines Polyethylenglycolethers eines linearen gesättigten oder ungesättigten C10-C18-Alkanols mit 1 - 5 Ethylenoxideinheiten im Molekül,
- insgesamt 0 - 1 Gew.-% mindestens eines Öls,
- bevorzugt mindestens ein organisches Lösemittel, ausgewählt aus ein- bis vierwertigen C2 - C6-Alkoholen, besonders bevorzugt in einer Gesamtmenge von 0,1 - 35 Gew.-%,
- optional 0,1 - 3 Gew.-% eines Aniontensids, ausgewählt aus Alkylsulfaten, Alkylethersulfaten und Ethercarbonsäuren mit 10 bis 20 C-Atomen in der Alkylgruppe und bis zu 16 Glykolethergruppen im Molekül, und
- gegebenenfalls mindestens ein Oxidationsfarbstoffvorprodukt enthält und einen pH-Wert im Bereich von 8 bis 11,5, gemessen bei 20°C, aufweist,
wobei alle Mengenangaben auf das Gewicht der Zusammensetzung (A) bezogen sind; Herstellung einer Mischung aus der vorgenannten Oxidationszusammensetzung (B) und der vorgenannten Zusammensetzung (A), bevorzugt im gewichtsbezogenen Mischungsverhältnis (A)/(B) im Bereich von 0,33 - 3, besonders bevorzugt 0,5 - 2, außerordentlich bevorzugt 1:1, unmittelbar danach Verteilung des anwendungsbereiten Mittels auf den Fasern,
Verbleib des Mittels auf den Fasern für einen Zeitraum von 1 bis 60 Minuten, unmittelbar danach
Ausspülen des verbliebenen Mittels aus den Fasern und ggf. Trocknen der Fasern.

## Claims

1. An oxidation composition for oxidative hair treatment, containing
- 50-96 wt.%, preferably 70-93 wt.%, particularly preferably 80-90 wt.%, water,
- 0.5-20 wt.% hydrogen peroxide,
- at least one linear saturated 1-alkanol having 12-30 carbon atoms, preferably in a total amount of 2-8 wt.%, particularly preferably 3 to 6.5 wt.%,
- at least one glyceryl fatty acid ester of general formula (I), 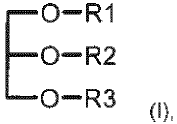 in which
- R1, R2 and R3 represent, independently of one another, a hydrogen atom or a grouping of formula (II), 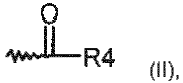 in which
- R4 represents an unbranched or branched, saturated or unsaturated C₁₁-C₂₇ alkyl group,
- with the proviso that at least one and at most two of the functional groups selected from R1, R2 and R3 represent a grouping of formula (II), the at least one glyceryl fatty acid ester of general formula (I) being contained preferably in a total amount of 0.01-1 wt.%, particularly preferably 0.1 to 0.8 wt.%,
- at least one ethoxylated fatty alcohol of formula (III) 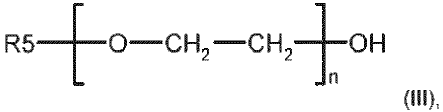 in which R5 represents a saturated or unsaturated, unbranched or branched C₈-C₂₄ alkyl group, preferably a saturated, unbranched C₁₆ or C₁₈ alkyl group, and n represents an integer from 10 to 30, preferably an integer from 12 to 25, more preferably an integer from 15 to 20, the at least one ethoxylated fatty alcohol of formula (III) being contained preferably in a total amount of 0.25-2 wt.%, particularly preferably 0.5 to 1.5 wt.%,
- at least one ethoxylated fatty alcohol of formula (IV) 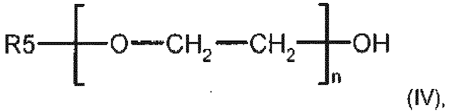 in which R5 represents a saturated or unsaturated, unbranched or branched C₈-C₂₄ alkyl group, preferably a saturated, unbranched C₁₆ or C₁₈ alkyl group, and n represents an integer from 50 to 150, preferably an integer from 80 to 120, more preferably the number 100, the at least one ethoxylated fatty alcohol of formula (IV) being contained preferably in a total amount of 0.03-0.3 wt.%, particularly preferably 0.08 to 0.25 wt.%, extremely preferably 0.1-0.2 wt.%,
- at least one polyol selected from C₂-C₉ alkanols having 2-6 hydroxyl groups and polyethylene glycols having 3-20 ethylene oxide units and mixtures thereof, preferably in a total amount of 1-10 wt.%, particularly preferably 2-8 wt.%, extremely preferably 3-6 wt.%, and
- at least one oil, preferably in a total amount of 0.1-5 wt.%, particularly preferably 0.2 to 3 wt.%, extremely preferably 0.5 to 2.5 wt.%,
all stated amounts being based on the weight of the oxidation composition, **characterized in that** at least one lamellar phase is contained.

2. The composition according to claim 1, **characterized in that** the at least one linear saturated 1-alkanol having 12-30 carbon atoms is selected from lauryl alcohol, myristyl alcohol, cetyl alcohol, stearyl alcohol, arachidyl alcohol, and behenyl alcohol and also from mixtures of these alkanols, preferably from cetyl alcohol, stearyl alcohol, arachidyl alcohol, behenyl alcohol and cetyl alcohol/stearyl alcohol mixtures.

3. The composition according to claim 1 or 2, **characterized in that** at least one compound selected from glyceryl monostearate, glyceryl distearate, glyceryl monopalmitate and glyceryl dipalmitate is contained, in a total amount of 0.01 to 1 wt.%, preferably 0.1 to 0.8 wt.%, based on the weight of the oxidation composition according to the invention, as the glyceryl fatty acid ester of formula (I).

4. The composition according to one of claims 1 to 3, **characterized in that** at least one compound selected from
- decan-1-ol (decyl alcohol), ethoxylated with 10 EO,
- decan-1-ol (decyl alcohol), ethoxylated with 20 EO,
- decan-1-ol (decyl alcohol), ethoxylated with 30 EO,
- dodecan-1-ol (dodecyl alcohol, lauryl alcohol), ethoxylated with 10 EO,
- dodecan-1-ol (dodecyl alcohol, lauryl alcohol), ethoxylated with 20 EO,
- dodecan-1-ol (dodecyl alcohol, lauryl alcohol), ethoxylated with 30 EO,
- hexadecan-1-ol (hexadecyl alcohol, cetyl alcohol, palmityl alcohol), ethoxylated with 10 EO,
- hexadecan-1-ol (hexadecyl alcohol, cetyl alcohol, palmityl alcohol), ethoxylated with 12 EO,
- hexadecan-1-ol (hexadecyl alcohol, cetyl alcohol, palmityl alcohol), ethoxylated with 15 EO,
- hexadecan-1-ol (hexadecyl alcohol, cetyl alcohol, palmityl alcohol), ethoxylated with 20 EO,
- hexadecan-1-ol (hexadecyl alcohol, cetyl alcohol, palmityl alcohol), ethoxylated with 25 EO,
- hexadecan-1-ol (hexadecyl alcohol, cetyl alcohol, palmityl alcohol), ethoxylated with 30 EO,
- octadecan-1-ol (octadecyl alcohol, stearyl alcohol), ethoxylated with 10 EO,
- octadecan-1-ol (octadecyl alcohol, stearyl alcohol), ethoxylated with 12 EO,
- octadecan-1-ol (octadecyl alcohol, stearyl alcohol), ethoxylated with 15 EO,
- octadecan-1-ol (octadecyl alcohol, stearyl alcohol), ethoxylated with 20 EO,
- octadecan-1-ol (octadecyl alcohol, stearyl alcohol), ethoxylated with 25 EO,
- octadecan-1-ol (octadecyl alcohol, stearyl alcohol), ethoxylated with 30 EO,
- eicosan-1-ol (eicosyl alcohol, arachidyl alcohol), ethoxylated with 10 EO,
- eicosan-1-ol (eicosyl alcohol, arachidyl alcohol), ethoxylated with 12 EO,
- eicosan-1-ol (eicosyl alcohol, arachidyl alcohol), ethoxylated with 15 EO,
- eicosan-1-ol (eicosyl alcohol, arachidyl alcohol), ethoxylated with 20 EO,
- eicosan-1-ol (eicosyl alcohol, arachidyl alcohol), ethoxylated with 25 EO,
- eicosan-1-ol (eicosyl alcohol, arachidyl alcohol), ethoxylated with 30 EO,
- docosan-1-ol (docosyl alcohol, behenyl alcohol), ethoxylated with 10 EO
- docosan-1-ol (docosyl alcohol, behenyl alcohol), ethoxylated with 12 EO
- docosan-1-ol (docosyl alcohol, behenyl alcohol), ethoxylated with 15 EO
- docosan-1-ol (docosyl alcohol, behenyl alcohol), ethoxylated with 20 EO
- docosan-1-ol (docosyl alcohol, behenyl alcohol), ethoxylated with 25 EO
- docosan-1-ol (docosyl alcohol, behenyl alcohol), ethoxylated with 30 EO,
and mixtures thereof is contained as the ethoxylated fatty alcohol of formula (III).

5. The composition according to one of claims 1 to 4, **characterized in that** at least one compound selected from
- hexadecan-1-ol (hexadecyl alcohol, cetyl alcohol, palmityl alcohol), ethoxylated with 50 EO,
- hexadecan-1-ol (hexadecyl alcohol, cetyl alcohol, palmityl alcohol), ethoxylated with 51 EO,
- hexadecan-1-ol (hexadecyl alcohol, cetyl alcohol, palmityl alcohol), ethoxylated with 52 EO,
- hexadecan-1-ol (hexadecyl alcohol, cetyl alcohol, palmityl alcohol), ethoxylated with 53 EO,
- hexadecan-1-ol (hexadecyl alcohol, cetyl alcohol, palmityl alcohol), ethoxylated with 54 EO,
- hexadecan-1-ol (hexadecyl alcohol, cetyl alcohol, palmityl alcohol), ethoxylated with 55 EO,
- hexadecan-1-ol (hexadecyl alcohol, cetyl alcohol, palmityl alcohol), ethoxylated with 56 EO,
- hexadecan-1-ol (hexadecyl alcohol, cetyl alcohol, palmityl alcohol), ethoxylated with 57 EO,
- hexadecan-1-ol (hexadecyl alcohol, cetyl alcohol, palmityl alcohol), ethoxylated with 58 EO,
- hexadecan-1-ol (hexadecyl alcohol, cetyl alcohol, palmityl alcohol), ethoxylated with 59 EO,
- hexadecan-1-ol (hexadecyl alcohol, cetyl alcohol, palmityl alcohol), ethoxylated with 60 EO,
- hexadecan-1-ol (hexadecyl alcohol, cetyl alcohol, palmityl alcohol), ethoxylated with 90 EO,
- hexadecan-1-ol (hexadecyl alcohol, cetyl alcohol, palmityl alcohol), ethoxylated with 91 EO,
- hexadecan-1-ol (hexadecyl alcohol, cetyl alcohol, palmityl alcohol), ethoxylated with 92 EO,
- hexadecan-1-ol (hexadecyl alcohol, cetyl alcohol, palmityl alcohol), ethoxylated with 93 EO,
- hexadecan-1-ol (hexadecyl alcohol, cetyl alcohol, palmityl alcohol), ethoxylated with 94 EO,
- hexadecan-1-ol (hexadecyl alcohol, cetyl alcohol, palmityl alcohol), ethoxylated with 95 EO,
- hexadecan-1-ol (hexadecyl alcohol, cetyl alcohol, palmityl alcohol), ethoxylated with 96 EO,
- hexadecan-1-ol (hexadecyl alcohol, cetyl alcohol, palmityl alcohol), ethoxylated with 97 EO,
- hexadecan-1-ol (hexadecyl alcohol, cetyl alcohol, palmityl alcohol), ethoxylated with 98 EO,
- hexadecan-1-ol (hexadecyl alcohol, cetyl alcohol, palmityl alcohol), ethoxylated with 99 EO,
- hexadecan-1-ol (hexadecyl alcohol, cetyl alcohol, palmityl alcohol), ethoxylated with 100 EO,
- hexadecan-1-ol (hexadecyl alcohol, cetyl alcohol, palmityl alcohol), ethoxylated with 101 EO,
- hexadecan-1-ol (hexadecyl alcohol, cetyl alcohol, palmityl alcohol), ethoxylated with 102 EO,
- hexadecan-1-ol (hexadecyl alcohol, cetyl alcohol, palmityl alcohol), ethoxylated with 103 EO,
- hexadecan-1-ol (hexadecyl alcohol, cetyl alcohol, palmityl alcohol), ethoxylated with 104 EO,
- hexadecan-1-ol (hexadecyl alcohol, cetyl alcohol, palmityl alcohol), ethoxylated with 105 EO,
- hexadecan-1-ol (hexadecyl alcohol, cetyl alcohol, palmityl alcohol), ethoxylated with 106 EO,
- hexadecan-1-ol (hexadecyl alcohol, cetyl alcohol, palmityl alcohol), ethoxylated with 107 EO,
- hexadecan-1-ol (hexadecyl alcohol, cetyl alcohol, palmityl alcohol), ethoxylated with 108 EO,
- hexadecan-1-ol (hexadecyl alcohol, cetyl alcohol, palmityl alcohol), ethoxylated with 109 EO,
- hexadecan-1-ol (hexadecyl alcohol, cetyl alcohol, palmityl alcohol), ethoxylated with 110 EO,
- octadecan-1-ol (octadecyl alcohol, stearyl alcohol), ethoxylated with 50 EO,
- octadecan-1-ol (octadecyl alcohol, stearyl alcohol), ethoxylated with 51 EO,
- octadecan-1-ol (octadecyl alcohol, stearyl alcohol), ethoxylated with 52 EO,
- octadecan-1-ol (octadecyl alcohol, stearyl alcohol), ethoxylated with 53 EO,
- octadecan-1-ol (octadecyl alcohol, stearyl alcohol), ethoxylated with 54 EO,
- octadecan-1-ol (octadecyl alcohol, stearyl alcohol), ethoxylated with 55 EO,
- octadecan-1-ol (octadecyl alcohol, stearyl alcohol), ethoxylated with 56 EO,
- octadecan-1-ol (octadecyl alcohol, stearyl alcohol), ethoxylated with 57 EO,
- octadecan-1-ol (octadecyl alcohol, stearyl alcohol), ethoxylated with 58 EO,
- octadecan-1-ol (octadecyl alcohol, stearyl alcohol), ethoxylated with 59 EO,
- octadecan-1-ol (octadecyl alcohol, stearyl alcohol), ethoxylated with 60 EO,
- octadecan-1-ol (octadecyl alcohol, stearyl alcohol), ethoxylated with 90 EO,
- octadecan-1-ol (octadecyl alcohol, stearyl alcohol) ethoxylated with 91 EO,
- octadecan-1-ol (octadecyl alcohol, stearyl alcohol), ethoxylated with 92 EO,
- octadecan-1-ol (octadecyl alcohol, stearyl alcohol), ethoxylated with 93 EO,
- octadecan-1-ol (octadecyl alcohol, stearyl alcohol), ethoxylated with 94 EO,
- octadecan-1-ol (octadecyl alcohol, stearyl alcohol), ethoxylated with 95 EO,
- octadecan-1-ol (octadecyl alcohol, stearyl alcohol), ethoxylated with 96 EO,
- octadecan-1-ol (octadecyl alcohol, stearyl alcohol), ethoxylated with 97 EO,
- octadecan-1-ol (octadecyl alcohol, stearyl alcohol), ethoxylated with 98 EO,
- octadecan-1-ol (octadecyl alcohol, stearyl alcohol), ethoxylated with 99 EO,
- octadecan-1-ol (octadecyl alcohol, stearyl alcohol), ethoxylated with 100 EO,
- octadecan-1-ol (octadecyl alcohol, stearyl alcohol), ethoxylated with 101 EO,
- octadecan-1-ol (octadecyl alcohol, stearyl alcohol), ethoxylated with 102 EO,
- octadecan-1-ol (octadecyl alcohol, stearyl alcohol), ethoxylated with 103 EO,
- octadecan-1-ol (octadecyl alcohol, stearyl alcohol), ethoxylated with 104 EO,
- octadecan-1-ol (octadecyl alcohol, stearyl alcohol), ethoxylated with 105 EO,
- octadecan-1-ol (octadecyl alcohol, stearyl alcohol), ethoxylated with 106 EO,
- octadecan-1-ol (octadecyl alcohol, stearyl alcohol), ethoxylated with 107 EO,
- octadecan-1-ol (octadecyl alcohol, stearyl alcohol), ethoxylated with 108 EO,
- octadecan-1-ol (octadecyl alcohol, stearyl alcohol), ethoxylated with 109 EO,
- octadecan-1-ol (octadecyl alcohol, stearyl alcohol), ethoxylated with 110 EO,
is contained as the ethoxylated fatty alcohol of formula (IV).

6. The composition according to one of claims 1 to 5, **characterized in that** the at least one polyol is selected from 1,2-propylene glycol, glycerol, butylene glycols such as 1,2-butylene glycol, 1,3-butylene glycol and 1,4-butylene glycol, pentylene glycols such as 1,2-pentanediol and 1,5-pentanediol, hexanediols such as 1,6-hexanediol and 2-methyl-1,3-propanediol (INCI: Hexylene glycol), hexanetriols such as 1,2,6-hexanetriol, 1,2-octanediol, 1,8-octanediol, dipropylene glycol, tripropylene glycol, diglycerol, triglycerol, erythritol, sorbitol, PEG-3, PEG-4, PEG-6, PEG-7, PEG-8, and mixtures of the above-mentioned substances.

7. The composition according to one of claims 1 to 6, **characterized in that** the at least one oil is selected from natural and synthetic hydrocarbons, particularly preferably from paraffin oils, C₁₈-C₃₀ isoparaffins, in particular isoeicosane, polyisobutene and polydecene, C₈-C₁₆ isoparaffins, and 1,3-di-(2-ethylhexyl)-cyclohexane; benzoic acid esters of linear or branched C₈₋₂₂ alkanols; fatty alcohols having 6-30 carbon atoms which are unsaturated or branched and saturated or branched and unsaturated; triglycerides of linear or branched, saturated or unsaturated, optionally hydroxylated C₈₋₃₀ fatty acids, in particular natural oils; dicarboxylic acid esters of linear or branched C₂-C₁₀ alkanols; esters of linear or branched, saturated or unsaturated fatty alcohols having 2-30 carbon atoms with linear or branched, saturated or unsaturated fatty acids having 2-30 carbon atoms, which may be hydroxylated; addition products of 1 to 5 propylene oxide units to monohydric or polyhydric C₈₋₂₂ alkanols; addition products of at least 6 ethylene oxide and/or propylene oxide units to monohydric or polyhydric C₃₋₂₂ alkanols; the C₈-C₂₂ fatty alcohol esters of monohydric or polyhydric C₂-C₇ hydroxycarboxylic acids; symmetrical, asymmetrical or cyclic esters of carbonic acid having C₃₋₂₂ alkanols, C₃₋₂₂ alkanediols or C₃₋₂₂ alkanetriols; esters of dimers of unsaturated C₁₂-C₂₂ fatty acids (dimer fatty acids) having monohydric linear, branched or cyclic C₂-C₁₈ alkanols or having polyhydric linear or branched C₂-C₆ alkanols; silicone oils, and mixtures of the above-mentioned substances.

8. A kit for oxidatively changing the color of keratin fibers, containing two separate compositions (A) and (B), wherein composition (B) is an oxidation composition according to one of claims 1-7 and composition (A) is in the form of a soap-based gel and contains
- 25-85 wt.% water,
- ammonia,
- 5-20 wt.%, preferably 8-16 wt.%, of at least one salt of a C12-22 fatty acid, preferably a salt of oleic acid,
- 2-20 wt.%, preferably 3-16 wt.%, of at least one polyethylene glycol ether of a linear saturated or unsaturated C10-C18 alkanol with 1-5 ethylene oxide units in the molecule,
- a total of 0-1 wt.% of at least one oil,
- preferably at least one organic solvent selected from monohydric to tetrahydric C2-C6 alcohols, particularly preferably in a total amount of 0.1-35 wt.%,
- optionally 0.1-3 wt.% of an anionic surfactant selected from alkyl sulfates, alkyl ether sulfates and ethercarboxylic acids having 10 to 20 C atoms in the alkyl group and up to 16 glycol ether groups in the molecule, and
- optionally at least one oxidation dye precursor
and has a pH in the range of 8 to 11.5, measured at 20 °C,
wherein all stated amounts are based on the weight of composition (A); wherein compositions (A) and (B) are present preferably in a weight-based ratio (A)/(B) in the range of 0.33-3, particularly preferably 0.5-2, extremely preferably 1:1.

9. A method for oxidatively changing the color of keratin fibers, **characterized by** the following method steps:
providing an oxidation composition (B) according to one of claims 1-7, containing
- 50-96 wt.%, preferably 70-93 wt.%, particularly preferably 80-90 wt.%, water,
- 0.5-20 wt.% hydrogen peroxide,
- at least one linear saturated 1-alkanol having 12-30 carbon atoms, preferably in a total amount of 2-8 wt.%, particularly preferably 3 to 6.5 wt.%,
- at least one glyceryl fatty acid ester of general formula (I), 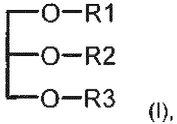 in which
- R1, R2 and R3 represent, independently of one another, a hydrogen atom or a grouping of formula (II), 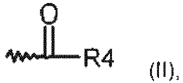 in which
- R4 represents an unbranched or branched, saturated or unsaturated C₁₁-C₂₇ alkyl group,
- with the proviso that at least one and at most two of the functional groups selected from R1, R2 and R3 represent a grouping of formula (II), the at least one glyceryl fatty acid ester of general formula (I) being contained preferably in a total amount of 0.01-1 wt.%, particularly preferably 0.1 to 0.8 wt.%,
- at least one ethoxylated fatty alcohol of formula (III) 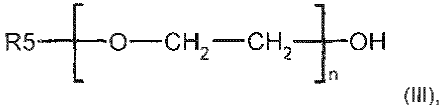 in which
R5 represents a saturated or unsaturated, unbranched or branched C₈-C₂₄ alkyl group, preferably a saturated, unbranched C₁₆ or C₁₈ alkyl group, and n represents an integer from 10 to 30, preferably an integer from 12 to 25, more preferably an integer from 15 to 20, the at least one ethoxylated fatty alcohol of formula (III) being contained preferably in a total amount of 0.25-2 wt.%, particularly preferably 0.5 to 1.5 wt.%,
- at least one ethoxylated fatty alcohol of formula (IV) 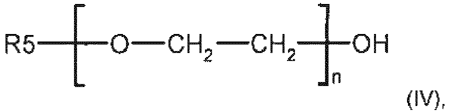 in which
R5 represents a saturated or unsaturated, unbranched or branched C₈-C₂₄ alkyl group, preferably a saturated, unbranched C₁₆ or C₁₈ alkyl group, and n represents an integer from 50 to 150, preferably an integer from 80 to 120, more preferably the number 100, the at least one ethoxylated fatty alcohol of formula (IV) being contained preferably in a total amount of 0.03-0.3 wt.%, particularly preferably 0.08 to 0.25 wt.%, extremely preferably 0.1-0.2 wt.%,
- at least one polyol selected from C₂-C₉ alkanols having 2-6 hydroxyl groups and polyethylene glycols having 3-20 ethylene oxide units and mixtures thereof, preferably in a total amount of 1-10 wt.%, particularly preferably 2-8 wt.%, extremely preferably 3-6 wt.%, and
- at least one oil, preferably in a total amount of 0.1-5 wt.%, particularly preferably 0.2 to 3 wt.%, extremely preferably 0.5 to 2.5 wt.%,
all stated amounts being based on the weight of the oxidation composition, **characterized in that** at least one lamellar phase is contained, and
providing a composition (A) which is in the form of a soap-based gel and contains
- 25-85 wt.% water,
- ammonia,
- 5-20 wt.%, preferably 8-16 wt.%, of at least one salt of a C12-22 fatty acid, preferably a salt of oleic acid,
- 2-20 wt.%, preferably 3-16 wt.%, of at least one polyethylene glycol ether of a linear saturated or unsaturated C10-C18 alkanol with 1-5 ethylene oxide units in the molecule,
- a total of 0-1 wt.% of at least one oil,
- preferably at least one organic solvent selected from monohydric to tetrahydric C2-C6 alcohols, particularly preferably in a total amount of 0.1-35 wt.%,
- optionally 0.1-3 wt.% of an anionic surfactant selected from alkyl sulfates, alkyl ether sulfates and ethercarboxylic acids having 10 to 20 C atoms in the alkyl group and up to 16 glycol ether groups in the molecule, and
- optionally at least one oxidation dye precursor
and has a pH in the range of 8 to 11.5, measured at 20 °C,
all stated amounts being based on the weight of composition (A);
preparing a mixture of the above-mentioned oxidation composition (B) and the above-mentioned composition (A), preferably in a weight-based mixing ratio (A)/(B) in the range of 0.33-3, particularly preferably 0.5-2, extremely preferably 1:1, and, directly thereafter, distributing the ready-to-use agent on the fibers, leaving the agent on the fibers for a period of 1 to 60 minutes and, directly thereafter, rinsing out the remaining agent from the fibers and optionally drying the fibers.

## Revendications

1. Composition d'oxydation pour le traitement capillaire oxydatif, contenant
- 50 à 96 % en poids, de préférence 70 à 93 % en poids, de manière particulièrement préférée 80 à 90 % en poids d'eau,
- 0,5 à 20 % en poids de peroxyde d'hydrogène,
- au moins un 1-alcanol linéaire saturé comportant 12 à 30 atomes de carbone, de préférence en une quantité totale de 2 à 8 % en poids, de manière particulièrement préférée de 3 à 6,5 % en poids,
- au moins un ester d'acide gras glycérylique de formule générale (I), 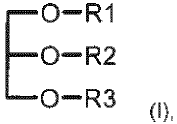 dans laquelle
- R1, R2 et R3 représentent chacun indépendamment un atome d'hydrogène ou un groupe de formule (II), 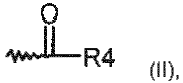 dans laquelle
- R4 représente un groupe alkyle C₁₁-C₂₇, ramifié ou non ramifié, saturé ou insaturé,
- à condition qu'au moins l'un et qu'au plus deux des radicaux choisis parmi R1, R2 et R3 représentent un groupe de formule (II), l'au moins un ester d'acide gras glycérylique de formule générale (I) étant présent de préférence en une quantité totale de 0,01 à 1 % en poids, de manière particulièrement préférée de 0,1 à 0,8 % en poids,
- au moins un alcool gras éthoxylé de formule (III) 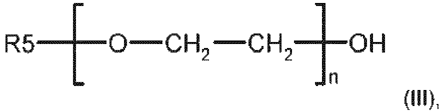 dans laquelle
R5 représente un groupe alkyle C₈-C₂₄ saturé ou insaturé, non ramifié ou ramifié, de préférence un groupe alkyle C₁₆- ou C₁₈- saturé, non ramifié, et n représente un nombre entier compris entre 10 et 30, de préférence un nombre entier compris entre 12 et 25, de manière davantage préférée un nombre entier compris entre 15 et 20, l'au moins un alcool gras éthoxylé de formule (III) étant présent de préférence en une quantité totale de 0,25 à 2 % en poids, de manière particulièrement préférée de 0,5 à 1,5 % en poids,
- au moins un alcool gras éthoxylé de formule (IV) 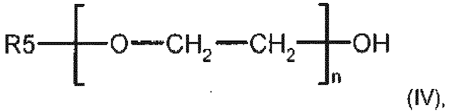 dans laquelle
R5 représente un groupe alkyle C₈-C₂₄ saturé ou insaturé, non ramifié ou ramifié, de préférence un groupe alkyle C₁₆- ou C₁₈- saturé, non ramifié, et n représente un nombre entier compris entre 50 et 150, de préférence un nombre entier compris entre 80 et 120, de manière davantage préférée le nombre 100, l'au moins un alcool gras éthoxylé de formule (IV) étant présent en une quantité totale de 0,03 à 0,3 % en poids d'alcool gras éthoxylé de formule (IV), de préférence de 0,08 à 0,25 % en poids, de manière exceptionnellement préférée de 0,1 à 0,2 % en poids,
- au moins un polyol choisi parmi les alcanols C₂-C₉ comportant 2 à 6 groupes hydroxyle et les polyéthylèneglycols comportant de 3 à 20 unités oxyde d'éthylène ainsi que des mélanges de ceux-ci, de préférence en une quantité totale de 1 à 10 % en poids, de préférence de 2 à 8 % en poids, de manière exceptionnellement préférée de 3 à 6 % en poids, et
- au moins une huile, de manière préférée en une quantité totale de 0, 1 à 5 % en poids, de manière particulièrement préférée de 0,2 à 3 % en poids, de manière exceptionnellement préférée de 0,5 à 2,5 % en poids,
toutes les quantités étant basées sur le poids de la composition d'oxydation, **caractérisée en ce qu'**au moins une phase lamellaire est présente.

2. Composition selon la revendication 1, **caractérisée en ce que** l'au moins un 1-alcanol saturé linéaire ayant de 12 à 30 atomes de carbone est choisi parmi l'alcool laurylique, l'alcool myristylique, l'alcool cétylique, l'alcool stéarylique, l'alcool arachidylique et l'alcool béhénylique, ainsi que les mélanges de ces alcanols, de préférence parmi l'alcool cétylique, l'alcool stéarylique, l'alcool arachidylique, l'alcool béhénylique et l'alcool cétylique, et les mélanges alcool cétylique/alcool stéarylique.

3. Composition selon la revendication 1 ou 2, **caractérisée en ce qu'**au moins un composé choisi parmi le monostéarate de glycéryle, le distéarate de glycéryle, le monopalmitate de glycéryle et le dipalmitate de glycéryle est présent sous forme d'ester d'acide gras de glycéryle de formule (I) en une quantité totale de 0,01 à 1 % en poids, de préférence de 0, 1 à 0,8 % en poids, par rapport au poids de la composition d'oxydation selon l'invention.

4. Composition selon l'une des revendications 1 à 3, **caractérisé en ce que**, comme alcool gras éthoxylé de formule (III), au moins un composé choisi parmi
- le décan-1-ol (alcool décylique), éthoxylé avec 10 EO,
- le décan-1-ol (alcool décylique), éthoxylé avec 20 EO,
- le décan-1-ol (alcool décylique), éthoxylé avec 30 EO,
- le dodécane-1-ol (alcool dodécylique, alcool laurique), éthoxylé avec 10 EO,
- le dodécane-1-ol (alcool dodécylique, alcool laurique), éthoxylé avec 20 EO,
- le dodécane-1-ol (alcool dodécylique, alcool laurique), éthoxylé avec 30 EO,
- l'hexadécane-1-ol (alcool hexadécylique, alcool cétylique, alcool palmitylique), éthoxylé avec 10 EO,
- l'hexadécane-1-ol (alcool hexadécylique, alcool cétylique, alcool palmitylique), éthoxylé avec 12 EO,
- l'hexadécane-1-ol (alcool hexadécylique, alcool cétylique, alcool palmitylique), éthoxylé avec 15 EO,
- l'hexadécane-1-ol (alcool hexadécylique, alcool cétylique, alcool palmitylique), éthoxylé avec 20 EO,
- l'hexadécane-1-ol (alcool hexadécylique, alcool cétylique, alcool palmitylique), éthoxylé avec 25 EO,
- l'hexadécane-1-ol (alcool hexadécylique, alcool cétylique, alcool palmitylique), éthoxylé avec 30 EO,
- l'octadécane-1-ol (alcool octadécylique, alcool stéarylique), éthoxylé avec 10 EO,
- l'octadécane-1-ol (alcool octadécylique, alcool stéarylique), éthoxylé avec 12 EO,
- l'octadécane-1-ol (alcool octadécylique, alcool stéarylique), éthoxylé avec 15 EO,
- l'octadécane-1-ol (alcool octadécylique, alcool stéarylique), éthoxylé avec 20 EO,
- l'octadécane-1-ol (alcool octadécylique, alcool stéarylique), éthoxylé avec 25 EO,
- l'octadécane-1-ol (alcool octadécylique, alcool stéarylique), éthoxylé avec 30 EO,
- l'éicosane-1-ol (alcool eicosylique, alcool arachylique), éthoxylé avec 10 EO,
- l'éicosane-1-ol (alcool eicosylique, alcool arachylique), éthoxylé avec 12 EO,
- l'éicosane-1-ol (alcool eicosylique, alcool arachylique), éthoxylé avec 15 EO,
- l'éicosane-1-ol (alcool eicosylique, alcool arachylique), éthoxylé avec 20 EO,
- l'éicosane-1-ol (alcool eicosylique, alcool arachylique), éthoxylé avec 25 EO,
- l'éicosane-1-ol (alcool eicosylique, alcool arachylique), éthoxylé avec 30 EO,
- le docosane-1-ol (alcool docosylique, alcool béhénylique), éthoxylé avec 10 EO
- le docosane-1-ol (alcool docosylique, alcool béhénylique), éthoxylé avec 12 EO
- le docosane-1-ol (alcool docosylique, alcool béhénylique), éthoxylé avec 15 EO
- le docosane-1-ol (alcool docosylique, alcool béhénylique), éthoxylé avec 20 EO
- le docosane-1-ol (alcool docosylique, alcool béhénylique), éthoxylé avec 25 EO
- le docosane-1-ol (alcool docosylique, alcool béhénylique), éthoxylé avec 30 EO, ainsi que des mélanges de ceux ci est présent.

5. Composition selon l'une des revendications 1 à 4, **caractérisé en ce que**, comme alcool gras éthoxylé de formule (IV), au moins un composé choisi parmi
- l'hexadécane-1-ol (alcool hexadécylique, alcool cétylique, alcool palmitylique), éthoxylé avec 50 EO,
- l'hexadécane-1-ol (alcool hexadécylique, alcool cétylique, alcool palmitylique), éthoxylé avec 51 EO,
- l'hexadécane-1-ol (alcool hexadécylique, alcool cétylique, alcool palmitylique), éthoxylé avec 52 EO,
- l'hexadécane-1-ol (alcool hexadécylique, alcool cétylique, alcool palmitylique), éthoxylé avec 53 EO,
- l'hexadécane-1-ol (alcool hexadécylique, alcool cétylique, alcool palmitylique), éthoxylé avec 54 EO,
- l'hexadécane-1-ol (alcool hexadécylique, alcool cétylique, alcool palmitylique), éthoxylé avec 55 EO,
- l'hexadécane-1-ol (alcool hexadécylique, alcool cétylique, alcool palmitylique), éthoxylé avec 56 EO,
- l'hexadécane-1-ol (alcool hexadécylique, alcool cétylique, alcool palmitylique), éthoxylé avec 57 EO,
- l'hexadécane-1-ol (alcool hexadécylique, alcool cétylique, alcool palmitylique), éthoxylé avec 58 EO,
- l'hexadécane-1-ol (alcool hexadécylique, alcool cétylique, alcool palmitylique), éthoxylé avec 59 EO,
- l'hexadécane-1-ol (alcool hexadécylique, alcool cétylique, alcool palmitylique), éthoxylé avec 60 EO,
- l'hexadécane-1-ol (alcool hexadécylique, alcool cétylique, alcool palmitylique), éthoxylé avec 90 EO,
- l'hexadécane-1-ol (alcool hexadécylique, alcool cétylique, alcool palmitylique), éthoxylé avec 91 EO,
- l'hexadécane-1-ol (alcool hexadécylique, alcool cétylique, alcool palmitylique), éthoxylé avec 92 EO,
- l'hexadécane-1-ol (alcool hexadécylique, alcool cétylique, alcool palmitylique), éthoxylé avec 93 EO,
- l'hexadécane-1-ol (alcool hexadécylique, alcool cétylique, alcool palmitylique), éthoxylé avec 94 EO,
- l'hexadécane-1-ol (alcool hexadécylique, alcool cétylique, alcool palmitylique), éthoxylé avec 95 EO,
- l'hexadécane-1-ol (alcool hexadécylique, alcool cétylique, alcool palmitylique), éthoxylé avec 96 EO,
- l'hexadécane-1-ol (alcool hexadécylique, alcool cétylique, alcool palmitylique), éthoxylé avec 97 EO,
- l'hexadécane-1-ol (alcool hexadécylique, alcool cétylique, alcool palmitylique), éthoxylé avec 98 EO,
- l'hexadécane-1-ol (alcool hexadécylique, alcool cétylique, alcool palmitylique), éthoxylé avec 99 EO,
- l'hexadécane-1-ol (alcool hexadécylique, alcool cétylique, alcool palmitylique), éthoxylé avec 100 EO,
- l'hexadécane-1-ol (alcool hexadécylique, alcool cétylique, alcool palmitylique), éthoxylé avec 101 EO,
- l'hexadécane-1-ol (alcool hexadécylique, alcool cétylique, alcool palmitylique), éthoxylé avec 102 EO,
- l'hexadécane-1-ol (alcool hexadécylique, alcool cétylique, alcool palmitylique), éthoxylé avec 103 EO,
- l'hexadécane-1-ol (alcool hexadécylique, alcool cétylique, alcool palmitylique), éthoxylé avec 104 EO,
- l'hexadécane-1-ol (alcool hexadécylique, alcool cétylique, alcool palmitylique), éthoxylé avec 105 EO,
- l'hexadécane-1-ol (alcool hexadécylique, alcool cétylique, alcool palmitylique), éthoxylé avec 106 EO,
- l'hexadécane-1-ol (alcool hexadécylique, alcool cétylique, alcool palmitylique), éthoxylé avec 107 EO,
- l'hexadécane-1-ol (alcool hexadécylique, alcool cétylique, alcool palmitylique), éthoxylé avec 108 EO,
- l'hexadécane-1-ol (alcool hexadécylique, alcool cétylique, alcool palmitylique), éthoxylé avec 109 EO,
- l'hexadécane-1-ol (alcool hexadécylique, alcool cétylique, alcool palmitylique), éthoxylé avec 110 EO,
- l'octadécane-1-ol (alcool octadécylique, alcool stéarylique), éthoxylé avec 50 EO,
- l'octadécane-1-ol (alcool octadécylique, alcool stéarylique), éthoxylé avec 51 EO,
- l'octadécane-1-ol (alcool octadécylique, alcool stéarylique), éthoxylé avec 52 EO,
- l'octadécane-1-ol (alcool octadécylique, alcool stéarylique), éthoxylé avec 53 EO,
- l'octadécane-1-ol (alcool octadécylique, alcool stéarylique), éthoxylé avec 54 EO,
- l'octadécane-1-ol (alcool octadécylique, alcool stéarylique), éthoxylé avec 55 EO,
- l'octadécane-1-ol (alcool octadécylique, alcool stéarylique), éthoxylé avec 56 EO,
- l'octadécane-1-ol (alcool octadécylique, alcool stéarylique), éthoxylé avec 57 EO,
- l'octadécane-1-ol (alcool octadécylique, alcool stéarylique), éthoxylé avec 58 EO,
- l'octadécane-1-ol (alcool octadécylique, alcool stéarylique), éthoxylé avec 59 EO,
- l'octadécane-1-ol (alcool octadécylique, alcool stéarylique), éthoxylé avec 60 EO,
- l'octadécane-1-ol (alcool octadécylique, alcool stéarylique), éthoxylé avec 90 EO,
- l'octadécane-1-ol (alcool octadécylique, alcool stéarylique), éthoxylé avec 91 EO,
- l'octadécane-1-ol (alcool octadécylique, alcool stéarylique), éthoxylé avec 92 EO,
- l'octadécane-1-ol (alcool octadécylique, alcool stéarylique), éthoxylé avec 93 EO,
- l'octadécane-1-ol (alcool octadécylique, alcool stéarylique), éthoxylé avec 94 EO,
- l'octadécane-1-ol (alcool octadécylique, alcool stéarylique), éthoxylé avec 95 EO,
- l'octadécane-1-ol (alcool octadécylique, alcool stéarylique), éthoxylé avec 96 EO,
- l'octadécane-1-ol (alcool octadécylique, alcool stéarylique), éthoxylé avec 97 EO,
- l'octadécane-1-ol (alcool octadécylique, alcool stéarylique), éthoxylé avec 98 EO,
- l'octadécane-1-ol (alcool octadécylique, alcool stéarylique), éthoxylé avec 99 EO,
- l'octadécane-1-ol (alcool octadécylique, alcool stéarylique), éthoxylé avec 100 EO,
- l'octadécane-1-ol (alcool octadécylique, alcool stéarylique), éthoxylé avec 101 EO,
- l'octadécane-1-ol (alcool octadécylique, alcool stéarylique), éthoxylé avec 102 EO,
- l'octadécane-1-ol (alcool octadécylique, alcool stéarylique), éthoxylé avec 103 EO,
- l'octadécane-1-ol (alcool octadécylique, alcool stéarylique), éthoxylé avec 104 EO,
- l'octadécane-1-ol (alcool octadécylique, alcool stéarylique), éthoxylé avec 105 EO,
- l'octadécane-1-ol (alcool octadécylique, alcool stéarylique), éthoxylé avec 106 EO,
- l'octadécane-1-ol (alcool octadécylique, alcool stéarylique), éthoxylé avec 107 EO,
- l'octadécane-1-ol (alcool octadécylique, alcool stéarylique), éthoxylé avec 108 EO,
- l'octadécane-1-ol (alcool octadécylique, alcool stéarylique), éthoxylé avec 109 EO,
- l'octadécane-1-ol (alcool octadécylique, alcool stéarylique), éthoxylé avec 110 EO, est présent.

6. Composition selon l'une des revendications 1 à 5, **caractérisé en ce qu'**au moins un polyol choisi parmi le 1,2-propylèneglycol, le glycérol, les butylèneglycols tels que le 1,2-butylèneglycol, le 1,3-butylèneglycol et le 1,4-butylèneglycol, les pentylèneglycols tels que le 1,2-pentanediol et le 1,5-pentanediol, les hexanediols tels que le 1,6-hexanediol et le 2-méthyl-1,3-propanediol (INCI : hexylènes glycol), les hexanetriols tels que le 1,2,6-hexanetriol, le 1,2-octanediol, le 1,8-octanediol, le dipropylène glycol, le tripropylène glycol, le diglycérol, le triglycérol, l'érythritol, le sorbitol, le PEG-3, le PEG-4, le PEG-6, le PEG-7 et le PEG-8, ainsi que des mélanges des substances précédentes.

7. Composition selon l'une des revendications 1 à 6, **caractérisé en ce que** l'au moins une huile est choisie parmi les hydrocarbures naturels et synthétiques, de manière particulièrement préférée parmi les huiles de paraffine, les isoparaffines C₁₈-C₃₀, en particulier l'isoeicosane, les polyisobutènes et les polydécènes, les isoparaffines C₈-C₁₆, ainsi que le 1,3-di-(2-ethylhexyl)-cyclohexane ;
les esters d'acides benzoïques d'alcanols C₈₋₂₂ linéaires ou ramifiés ;
les alcools gras ayant de 6 à 30 atomes de carbone qui sont insaturés ou ramifiés et saturés ou ramifiés et insaturés ;
les triglycérides d'acides gras C₈₋₃₀ linéaires ou ramifiés, saturés ou insaturés, éventuellement hydroxylés, en particulier les huiles naturelles ;
les esters dicarboxyliques d'alcanols C_{2-C10} linéaires ou ramifiés ;
les esters d'alcools gras saturés ou insaturés, linéaires ou ramifiés, ayant de 2 à 30 atomes de carbone avec des acides gras saturés ou insaturés, linéaires ou ramifiés, ayant de 2 à 30 atomes de carbone, qui peuvent être hydroxylés ;
les produits d'addition de 1 à 5 unités d'oxyde de propylène aux alcanols C₈₋₂₂ monovalents ou polyvalents ;
les produits d'addition d'au moins 6 unités d'oxyde d'éthylène et/ou d'oxyde de propylène aux alcanoles C₃₋₂₂ monovalents ou polyvalents ;
les esters d'alcools gras C_{8-C22} d'acides hydroxycarboxyliques C_{C2-C7} monovalents ou polyvalents ;
les esters symétriques, asymétriques ou cycliques de l'acide carbonique comportant des alcanols C₃₋₂₂, alcanediols C₃₋₂₂ ou alcanetriols C₃₋₂₂ ;
les esters de dimères d'acides gras C₁₂-C₂₂ insaturés (acides gras dimères) avec des alcanols C₂-C₁₈ monovalents linéaires, ramifiés ou cycliques ou avec des alcanols C₂-C₆ polyvalents, linéaires ou ramifiés ;
les huiles de silicone ainsi que des mélanges des substances précédentes.

8. Kit de changement de couleur oxydatif des fibres kératiniques, comprenant deux compositions séparées (A) et (B), la composition (B) étant une composition d'oxydation selon l'une des revendications 1 à 7 et la composition (A) se présentant sous la forme d'un gel à base de savon, et contient
- 25 à 85 % en poids d'eau,
- l'ammoniaque,
- 5 à 20 % en poids, de préférence 8 à 16 % en poids d'au moins un sel d'un acide gras C12-C22, de préférence un sel d'acide oléique,
- 2 à 20 % en poids, de préférence 3 à 16 % en poids d'au moins un polyéthylèneglycol éther d'un alcanol linéaire saturé ou insaturé C10-C18 comportant 1 à 5 unités d'oxyde d'éthylène dans la molécule 0 à 1 % en poids d'au moins une huile,
- de préférence au moins un solvant organique choisi parmi les alcools C2 - C6 mono à tétravalents, de manière particulièrement préférée en une quantité totale de 0,1 à 35 % en poids,
- éventuellement 0,1 à 3 % en poids d'un tensioactif anionique choisi parmi les sulfates d'alkyle, les sulfates d'alkyléther et les acides éthercarboxyliques ayant 10 à 20 atomes de carbone dans le groupe alkyle et jusqu'à 16 groupes éther de glycol dans la molécule, et
- éventuellement au moins un précurseur de colorant par oxydation et un pH dans la plage compris entre 8 et 11,5, mesuré à 20°C, toutes les quantités étant basées sur le poids de la composition (A) ;
les compositions (A) et (B) étant de préférence présentes dans un rapport pondéral (A)/(B) dans la plage compris entre 0,33 et 3, de manière particulièrement préférée entre 0,5 et 2, et étant de manière extraordinairement préférée de 1:1.

9. Procédé de changement de couleur oxydatif des fibres kératiniques, **caractérisé par** les étapes de procédé suivantes :
fourniture d'une composition d'oxydation (B) selon l'une des revendications 1 à 7, contenant
- 50 à 96 % en poids, de préférence 70 à 93 % en poids, de manière particulièrement préférée 80 à 90 % en poids d'eau,
- 0,5 à 20 % en poids de peroxyde d'hydrogène,
- au moins un 1-alcanol linéaire saturé comportant 12 à 30 atomes de carbone, de préférence en une quantité totale de 2 à 8 % en poids, de manière particulièrement préférée de 3 à 6,5 % en poids,
- au moins un ester d'acide gras glycérylique de formule générale (I), 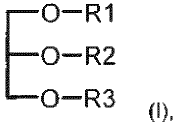 dans laquelle
- R1, R2 et R3 représentent chacun indépendamment un atome d'hydrogène ou un groupe de formule (II), 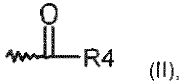 dans laquelle
- R4 représente un groupe alkyle C₁₁-C₂₇, ramifié ou non ramifié, saturé ou insaturé,
- à condition qu'au moins l'un et qu'au plus deux des radicaux choisis parmi R1, R2 et R3 représentent un groupe de formule (II), l'au moins un ester d'acide gras glycérylique de formule générale (I) étant présent de préférence en une quantité totale de 0,01 à 1 % en poids, de manière particulièrement préférée de 0,1 à 0,8 % en poids,
- au moins un alcool gras éthoxylé de formule (III) 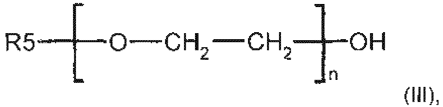 dans laquelle
R5 représente un groupe alkyle C₈-C₂₄ saturé ou insaturé, non ramifié ou ramifié, de préférence un groupe alkyle C₁₆- ou C₁₈- saturé, non ramifié, et n représente un nombre entier compris entre 10 et 30, de préférence un nombre entier compris entre 12 et 25, de manière davantage préférée un nombre entier compris entre 15 et 20, l'au moins un alcool gras éthoxylé de formule (III) étant présent de préférence en une quantité totale de 0,25 à 2 % en poids, de manière particulièrement préférée de 0,5 à 1,5 % en poids,
- au moins un alcool gras éthoxylé de formule (IV) 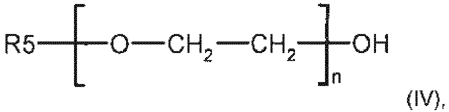 dans laquelle
R5 représente un groupe alkyle C₈-C₂₄ saturé ou insaturé, non ramifié ou ramifié, de préférence un groupe alkyle C₁₆- ou C₁₈- saturé, non ramifié, et n représente un nombre entier compris entre 50 et 150, de préférence un nombre entier compris entre 80 et 120, de manière davantage préférée le nombre 100, l'au moins un alcool gras éthoxylé de formule (IV) étant présent en une quantité totale de 0,03 à 0,3 % en poids d'alcool gras éthoxylé de formule (IV), de préférence de 0,08 à 0,25 % en poids, de manière exceptionnellement préférée de 0,1 à 0,2 % en poids,
- au moins un polyol choisi parmi les alcanols C₂-C₉ comportant 2 à 6 groupes hydroxyle et les polyéthylèneglycols comportant de 3 à 20 unités oxyde d'éthylène ainsi que des mélanges de ceux-ci, de préférence en une quantité totale de 1 à 10 % en poids, de préférence de 2 à 8 % en poids, de manière exceptionnellement préférée de 3 à 6 % en poids, et
- au moins une huile, de manière préférée en une quantité totale de 0,1 à 5 % en poids, de manière particulièrement préférée de 0,2 à 3 % en poids, de manière exceptionnellement préférée de 0,5 à 2,5 % en poids,
toutes les quantités étant basées sur le poids de la composition d'oxydation, **caractérisé en ce qu'**au moins une phase lamellaire est présente, et fourniture d'une composition (A) qui se présente sous la forme d'un gel à base de savon, et
- 25 à 85 % en poids d'eau,
- l'ammoniaque,
- 5 à 20 % en poids, de préférence 8 à 16 % en poids d'au moins un sel d'un acide gras C12-C22, de préférence un sel d'acide oléique,
- 2 à 20 % en poids, de préférence 3 à 16 % en poids, d'au moins un polyéthylèneglycol éther d'un alcanol linéaire saturé ou insaturé C10-C18 comportant 1 à 5 unités oxyde d'éthylène dans la molécule,
- au total 0 à 1 % en poids d'au moins une huile,
- de préférence au moins un solvant organique choisi parmi les alcools C2 - C6 mono à tétravalents, de manière particulièrement préférée en une quantité totale de 0,1 à 35 % en poids,
- éventuellement 0,1 à 3 % en poids d'un tensioactif anionique choisi parmi les sulfates d'alkyle, les sulfates d'alkyléther et les acides éthercarboxyliques ayant 10 à 20 atomes de carbone dans le groupe alkyle et jusqu'à 16 groupes éther de glycol dans la molécule, et
- éventuellement au moins un précurseur de colorant par oxydation
et un pH dans la plage compris entre 8 et 11,5, mesuré à 20°C, toutes les quantités étant basées sur le poids de la composition (A) ;
Préparation d'un mélange de la composition d'oxydation (B) précédentes et de la composition (A) précédentes, de préférence dans le rapport de mélange (A)/(B) en fonction du poids dans la plage comprise entre 0,33 et 3, de manière particulièrement préférée entre 0,5 à 2, de manière extraordinairement préférée de 1:1, immédiatement après distribution de l'agent prêt à être appliqué sur les fibres, rétention de l'agent sur les fibres pendant 1 à 60 minutes, puis rinçage immédiat du reste d'agent des fibres et éventuellement séchage des fibres.
